Europäisches Patentamt

European Patent Office

Office européen des brevets

Veröffentlichungsnummer: **0 525 610 A2**

## EUROPÄISCHE PATENTANMELDUNG

Anmeldenummer: **92112509.2**

Anmeldetag: **22.07.92**

Int. Cl.5: **C12N 15/57**, C12N 9/54, C12N 9/20, C12N 9/24, C11D 3/386

Priorität: **27.07.91 DE 4124997**

Veröffentlichungstag der Anmeldung: **03.02.93 Patentblatt 93/05**

Benannte Vertragsstaaten: **AT BE CH DE DK FR GB IT LI NL SE**

Anmelder: **Solvay Enzymes GmbH & Co. KG Grosse Drakenburger Strasse 95 W-3070 Nienburg/Weser(DE)** Anmelder: **Gesellschaft für Biotechnologische Forschung mbH (GBF) Mascheroder Weg 1 W-3300 Braunschweig-Stöckheim(DE)**

Erfinder: **Vetter, Roman Warneckeweg 1 W-3167 Burgdorf(DE)** Erfinder: **Mücke, Ingo Knickstrasse 63A W-3013 Barsinghausen(DE)** Erfinder: **Wilke, Detlef**

Landwehr 6 W-3015 Wennigsen(DE) Erfinder: **Amory, Antoine Avenue Bel Air, 44 B-1330 Rixensart(BE)** Erfinder: **Aehle, Wolfgang Lumumbasingel 7 NL-2622 ED Delft(DE)** Erfinder: **Sobek, Harald Friedrich-Schiller-Strasse 24 W-7951 Ummendorf(DE)** Erfinder: **Schomburg, Dietmar Lübeckstrasse 50 W-3300 Braunschweig(DE)** Erfinder: **Clippe, André Avenue Slegers, 207 B-1200 Bruxelles(BE)**

Vertreter: **Lauer, Dieter, Dr. c/o Solvay Deutschland GmbH, Postfach 220, Hans-Böckler-Allee 20 W-3000 Hannover 1(DE)**

Verfahren zur Verbesserung der Stabilität von Enzymen und stabilisierte Enzyme.

Beschrieben wird die Stabilisierung von Enzymen gegen destabilisierende Einwirkung von ionischen Tensiden und derart stabilisierte Enzyme. Zur Stabilisierung werden DNA-Sequenzen, die für die Enzyme codieren, in bestimmten Positionen, die zu bestimmten Oberflächenbereichen des Enzyms korrelieren, derart durch gerichtete Mutation verändert, daß das Codon, in welchem sich die Mutation befindet, nunmehr für eine gegenüber der ursprünglichen Aminosäure andere Aminosäure codiert.

Die vorliegende Erfindung bezieht sich auf ein Verfahren zur Verbesserung der Stabilität von Enzymen gegenüber ionischen Tensiden durch gerichtete Mutagenese von für diese Enzyme codierenden DNA-Sequenzen und nachfolgende Expression dieser Enzyme mit verbesserter ionischer Tensidstabilität.

Enzyme wie Proteasen, Lipasen, Amylasen, Cellulasen etc. sind wertvolle industrielle Produkte mit vorteilhaften Anwendungen in der Waschmittelindustrie, da sie z. B. enzymatisch spaltbare Verunreingungen abbauen und somit eine leichtere Entfernung dieser Verunreinigungen ermöglichen. Um wirksam zu sein, müssen diese Enzyme nicht nur enzymatische Aktivität unter Waschbedingungen (pH-Wert, Temperatur) besitzen, sondern müssen darüber hinaus auch mit anderen Waschmittelbestandteilen, insbesondere z.B. in Kombination mit Tensiden, verträglich sein, d.h. in Gegenwart dieser Substanzen ausreichende Stabilität und ausreichende Wirksamkeit aufweisen. Hierbei können insbesondere die in Wasch- und Reinigungsmittelzusammensetzungen häufig verwendeten Tenside ionischen Typs die Stabilität der verwendeten Waschmittelenzyme negativ beeinflussen, so daß die Aktivität der Enzyme in Gegenwart des ionischen Tensids rasch absinkt und die Aktivität selbst bei kurzen Waschzyklen von etwa 30 Minuten kaum ausreichend genutzt werden kann. Die auf die mangelnde Stabilität der Enzyme gegenüber ionischen Tensiden zurückzuführende unzureichende Ausnutzung der enzymatischen Aktivität während des Wasch- bzw. Reinigungsvorganges bedingt somit auch deutlich verminderte Wasch- bzw. Reinigungsleistungen. Eine gute Stabilität der Wasch- und Reinigungsmittelenzyme gegenüber ionischen Tensiden ist insbesondere für Flüssigformulierungen von Wasch- und Reinigungsmitteln erforderlich, da die Enzyme in diesen Formulierungen nicht wie in Pulverformulierungen durch Beschichtungsverfahren gegen destabilisierende Einwirkungen anderer Formulierungskomponenten, wie insbesondere ionische Tenside, geschützt werden können.

Es bestand daher die Aufgabe, Enzyme mit einer guten Stabilität gegenüber ionischen, insbesondere anionischen, Tensiden bereitzustellen und ein hierfür geeignetes Verfahren anzugeben.

Es wurde gefunden, daß in Enzymen durch Austausch von Aminosäuren in einem hydrophoben Oberflächenbereich des Enzyms eine gute Stabilität gegenüber ionischen Tensiden erzielt werden kann.

Gegenstand der Erfindung sind gegen destabilisierende Einwirkungen von ionischen Tensiden stabilisierte Enzyme, bei denen wenigstens eine der Aminosäuren, die sich in einem hydrophoben Oberflächenbereich des Enzyms oder in direkter Nachbarschaft zu diesem hydrophoben Oberflächenbereich befinden, durch eine andere Aminosäure ausgetauscht ist, wobei

(a) eine hydrophobe Aminosäure, deren Aminosäurerest an der Bildung eines hydrophoben Oberflächenbereiches beteiligt ist, in dessen räumlicher Nachbarschaft sich wenigstens eine Aminosäure mit ionischem Aminosäurerest befindet, durch eine andere Aminosäure mit hydrophilem Aminosäurerest ausgetauscht ist, und/oder wobei

(b) eine polare, ungeladene Aminosäure, die an einen hydrophoben Oberflächenbereich angrenzt, in dessen räumlicher Nachbarschaft sich wenigstens eine Aminosäure mit ionischem Aminosäurerest befindet, durch eine gegenüber der ursprünglichen Aminosäure sterisch anspruchvollere Aminosäure, vorzugsweise durch eine sterisch anspruchvollere Aminosäure mit einem langkettigeren hydrophilen Aminosäurerest, ausgetauscht ist, und/oder wobei

(c) eine ionische Aminosäure, die sich in räumlicher Nachbarschaft zu einem hydrophoben Oberflächenbereich befindet, durch eine Aminosäure mit einem ungeladenen hydrophilen Aminosäurerest oder durch eine geladene Aminosäure, deren Aminosäurerest eine zum ionischen Tensid gleichgerichtete Ladung aufweist, ausgetauscht ist.

In einer zweckmäßigen Ausgestaltung der Erfindung liegen Enzyme vor, in denen die ursprüngliche Aminosäure gemäß (a), (b) und/oder (c) in bzw. bei einem solchen hydrophoben Oberflächenbereich des Enzyms ausgetauscht ist, der auf der Enzymoberfläche als eine Vertiefung, insbesondere als eine Mulde, eine Senke oder ein in das Enzyminnere eindringendes Loch, ausgebildet ist.

Ohne hier eine bestimmte Theorie festlegen zu wollen, kann angenommen werden, daß geladene Gruppen von ionischen Tensiden durch elektrostatisch entgegengesetzt geladene Bereiche auf der Enzymoberfläche angezogen werden und durch elektrostatische Kräfte eine erste Bindung mit dem Enzym eingehen. Es kann wahrscheinlich angenommen werden, daß nach dieser ersten Bindung des ionischen Tensids der langkettige unpolare Rest des Tensidmoleküls in enge Wechselwirkung mit zugänglichen hydrophoben Oberflächenbereichen des Enzyms treten kann, die sich in direkter Nachbarschaft zur elektrostatischen Bindungsstelle des ionischen Tensids befinden. Die Wechselwirkungen zwischen dem apolaren Tensidrest und dem hydrophen Oberflächenbereich des Enzyms kann zur Denaturierung des Enzyms und damit zu dessen Desaktivierung führen, insbesondere dann, wenn der hydrophobe Bereich als Mulde, Senke oder Loch ausgebildet ist. Hierdurch wird dem apolaren Tensidrest der Zugang in den hydrophoben Kern des Enzyms ermöglichst, sofern dem apolaren Tensidrest der Weg in das Enzyminnere nicht durch stabile Strukturelemente des Enzyms wie beispielsweise eine Helix oder eine rigide Faltung etc.

versperrt wird. Kann der apolare Tensidrest, insbesondere im Falle von hydrophoben Mulden, Senken oder Löchern, über den hydrophoben Oberflächenbereich des Enzyms leicht in das Enzyminnere eindringen, so ist die Auffaltung und des Desaktivierung des Enzyms wahrscheinlich.

In bevorzugten stabilisierten Enzymen der Erfindung ist die Stabilität des Enzyms gegen destabilisierende Einwirkung von anionischen Tensiden durch einen Aminosäureaustausch gemäß (a) oder (b) oder durch einen Austausch einer kationischen Aminosäure durch eine Aminosäure mit einem ungeladenen hydrophilen oder mit einem anionischen Aminosäurerest verbessert.

Die erfindungsgemäßen stabilisierten Enzyme können an sich jedes in der Wasch- und Reinigungsmittelindustrie einsetzbare Enzym umfassen, beispielsweise Enzyme wie Proteasen, Lipasen, Amylasen, Pullulanasen, Glucanasen, Pektinasen, Nukleasen, Oxidoreduktasen etc. Zweckmäßig sind als stabilisierte Enzyme insbesondere Proteasen, Lipasen, Amylasen oder Cellulasen, wobei gegen destabilisierende Einwirkungen von ionischen Tensiden stabilisierte Proteasen in einer Ausgestaltung der Erfindung bevorzugt sind. Im Folgenden wird daher die Erfindung stellvertretend für die vorstehend genannten Enzyme am Beispiel der Proteasen eingehender beschrieben. Als Proteasen sind insbesondere die sogenannten Subtilisine vorteilhaft. Subtilisine sind alkalische Serinproteasen, d.h. Proteasen mit pH-Optimum im alkalischen pH-Bereich und mit einem essentiellen Serinrest im aktiven Zentrum. Die Subtilisine der vorliegenden Erfindung können durch Kultivierung von Gram-positiven Bakterien oder Pilzen gewonnen werden. Sehr bekannte Subtilisine des Standes der Technik werden aus Bacillus-Stämmen gewonnen, beispielsweise Subtilisine wie Subtilisin BPN' oder Subtilisin Carlsberg. Hierzu gehören auch alkalische Proteasen, die durch Kultivierung von Bacillus subtilis, Bacillus amyloliquefaciens, Bacillus licheniformis oder Bacillus lentus gewonnen werden können. Ganz besonders bevorzugte Subtilisine sind die hochalkalischen Serinproteasen, die insbesondere durch Kultivierung von Bacillus-Spezies wie z.B. Bacillus alcalophilus gewonnen werden können.

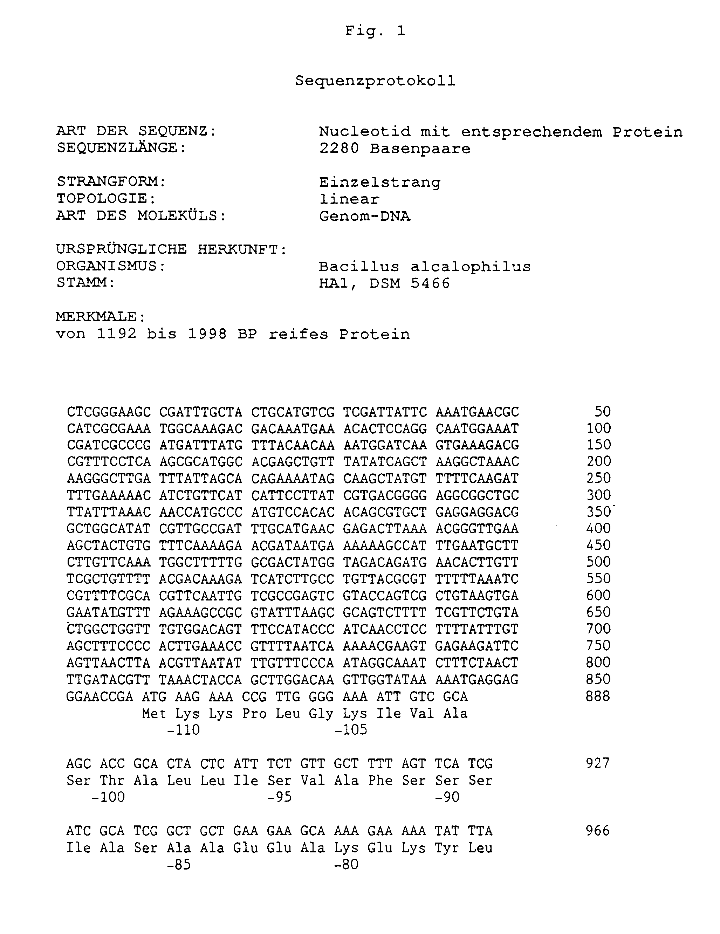

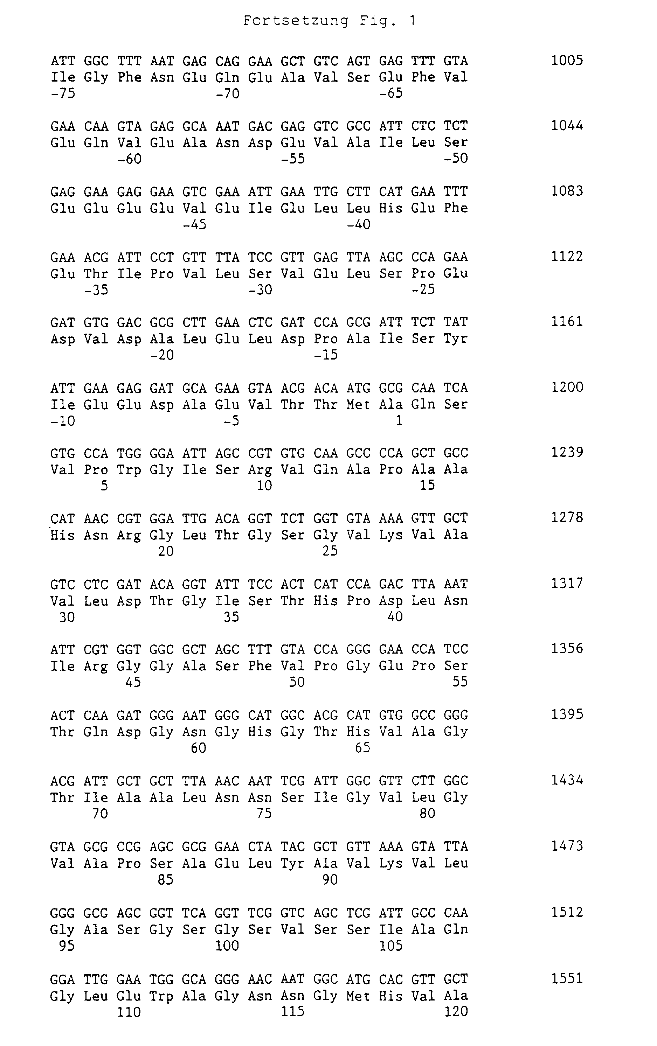

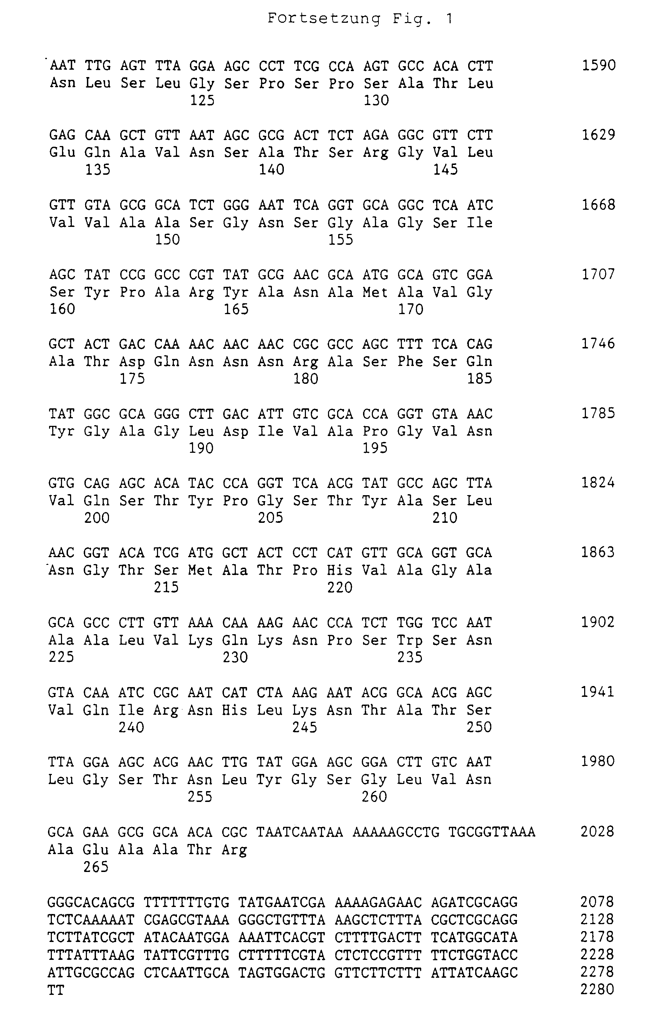

In einer speziellen Ausgestaltung der Erfindung besitzen diese hochalkalischen Proteasen eine Aminosäurensequenz mit mindestens 80 %, vorzugsweise mindestens 90 %, insbesondere aber mindestens 95 % Homologie zu der in Fig. 1 angegebenen Aminosäurensequenz, wobei in der Aminosäurensequenz der hochalkalischen Protease wenigstens eine der ursprünglichen Aminosäuren wie oben unter (a), (b) und/oder (c) angegeben gegen eine andere Aminosäure ausgetauscht ist.

Unter Homologie zu der in Fig. 1 angebenen Aminosäurensequenz wird hier die strukturelle Verwandtschaft der betreffenden Aminosäurensequenzen zu der in Fig. 1 angegebenen Aminosäurensequenz verstanden. Zur Bestimmung der Homologie werden jeweils die einander strukturell entsprechenden Abschnitte der Aminosäurensequenz der Fig. 1 und der damit zu vergleichenden Aminosäurensequenz so zur Deckung miteinander gebracht, daß maximale Strukturübereinstimmung zwischen den Aminosäurensequenzen besteht, wobei durch Deletion oder Insertion einzelner Aminosäuren verursachte Unterschiede berücksichtigt und durch entsprechende Verschiebungen von Sequenzabschnitten ausgeglichen werden. Die Zahl der nunmehr in den Sequenzen miteinander übereinstimmenden Aminosäuren ("homologe Positionen") bezogen auf die Gesamtzahl der in der Sequenz der Fig. 1 enthaltenen Aminosäuren gibt dabei die Homologie in % an. Abweichungen in den Sequenzen können sowohl durch Variation, Insertion als auch Deletion von Aminosäuren bedingt sein.

Es versteht sich dementsprechend von selbst, daß bei Einsatz von zur Figur 1 wenigstens zu 80 % homologen hochalkalischen Proteasen, die mit Bezug auf die Fig. 1 bezeichneten Aminosäurenpositionen sich auf die dazu homologen Positionen der jeweils eingesetzten Protease beziehen. Deletionen oder Insertionen in den Aminosäurensequenzen der zu Fig. 1 homologen Proteasen können zu einer relativen Verschiebung der Aminosäurenpositionen führen, so daß in homologen Teilstücken von zueinander homologen Aminosäurensequenzen die numerischen Bezeichnungen der einander entsprechenden Aminosäurepositonen nicht identisch zu sein brauchen.

Zweckmäßig stabilisierte hochalkalische Proteasen der Erfindung sind insbesondere solche, die durch Austauschen von Aminosäuren in wenigstens einer der Positionen 4, 14, 27, 39, 43, 47, 49, 77, 80, 89, 111, 117, 118, 127, 143, 161, 164, 165, 208, 232, 233, 235, 237, 249 oder 256, vorzugsweise 27, 43, 118, 143, 164, 237, oder 249, der Fig. 1 oder in einer der dazu homologen Positionen stabilisiert sind. Von den vorstehenden Austauschpositionen sind insbesondere die Positionen 27, 43, 118, 143, 164, 237 und 249 der Fig. 1 sowie die dazu homologen Postitionen vorteilhaft. Beispiele für besonders bevorzugt stabilisierte hochalkalische Proteasen sind solche, die durch wenigstens einen der Aminosäureaustausche K27Q, I43R, I43K, I43Q, I43E, H118W, H118Y, R143N, R143S, R143T, R164Q, N237P, T249R, T249K, T249Q und T249E, mit den auf Fig. 1 bezogenen Positionen oder einen hierzu homologen Aminosäureaustausch stabilisiert sind. Für die Bezeichnung der Mutationen wird hier die im Stand der Technik übliche Nomenklatur verwendet. Die Aminosäuren werden durch den Einbuchstabencode bezeichnet, wobei die ursprüngliche Aminosäure der Positionsangabe vorangestellt und die zur Stabilisierung eingeführte Aminosäure der Positionsangabe nachgestellt ist.

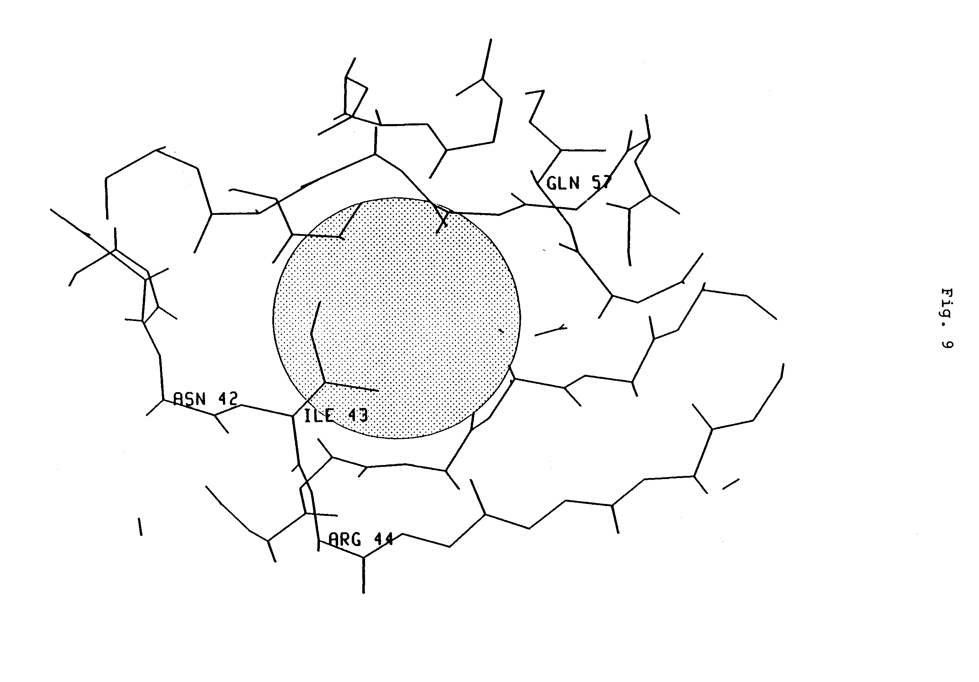

Gemäß Variante (a) der Erfindung ist in den Enzymen eine hydrophile Aminosäure anstelle einer hydrophoben Aminosäure in einen apolaren Bereich der Enzymoberfläche eingeführt. Die ausgetauschten hydrophoben Aminosäuren im Sinne der Erfindung können Glycin (= Gly, G), Alanin (= Ala, A), Valin (= Val, V), Leucin (= Leu, L), Isoleucin (= Ile, I), Phenylalanin (= Phe, F), Prolin (= Pro, P) sein; insbesondere die Aminosäuren Alanin, Valin, Leucin und Isoleucin. Die an die Stelle der ursprünglichen, hydrophoben Aminosäure tretende neue, hydrophile Aminosäure kann sowohl einen geladenen als auch einen ungeladenen polaren Aminosäurerest besitzen. Hydrophile Aminosäuren mit geladenem Aminosäurerest sind z.B. die Aminosäuren Asparaginsäure (= Asp, D), Glutaminsäure (= Glu, E), Lysin (= Lys, K), Arginin (= Arg, R); hydrophile Aminosäuren mit einem ungeladenen polaren Aminosäurerest sind insbesondere z.B. Asparagin (= Asn, N) und Glutamin (= Gln, Q). Bevorzugt sind insbesondere die langkettigeren Aminosäuren Glutamin, Glutaminsäure, Arginin und Lysin. Ein Beispiel für einen hydrophoben Bereich, in dem ein Aminosäureaustausch gemäß (a) zu einer stabilisierten hochalkalischen Protease aus Bacillus alcalophilus führt, ist der in Fig. 9 dargestellte hydrophobe Bereich der Aminosäureposition Ile 43. Dieser hydrophobe Bereich ist durch die polaren Aminosäuren Gln 57, Asn 42 und die ionische Aminosäure Arg 44 umgeben. In diesem hydrophoben Bereich führt der Ersatz von Ile 43 beispielsweise durch Arginin, Lysin, Glutamin oder Glutaminsäure zu erfindungsgemäß stabilisierten hochalkalischen Proteasen mit einer der Mutationen I43R, I43K, I43Q und I43E.

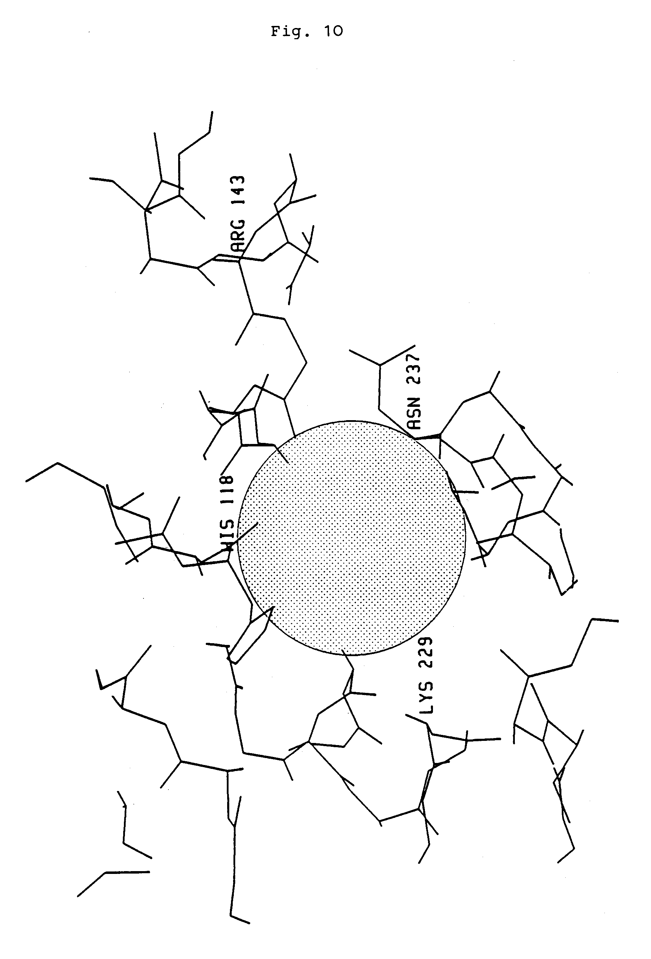

Gemäß Variante (b) der Erfindung ist in den Enzymen anstelle einer polaren ungeladenen, an den apolaren Bereich der Enzymoberfläche angrenzenden Aminosäure eine gegenüber dieser ursprünglichen Aminosäure sterisch anspruchsvollere Aminosäure eingeführt. Die auszutauschenden polaren ungeladenen Aminosäuren im Sinne dieser Variante der Erfindung sind bspw. die Aminosäuren Tyrosin, und insbesondere Threonin, Asparagin und Histidin. Für den Austausch der ursprünglichen Aminosäure gemäß Variante (b) der Erfindung ist an sich jede gegenüber der ursprünglichen Aminosäure sterisch genügend anspruchsvolle Aminosäure, ggf. sogar hydrophobe Aminosäure, geeignet. Durch die sterisch anspruchsvollere Aminosäure wird der apolare Bereich gegen die destabilisierende Einwirkung des lipophilen Tensidrestes abgeschirmt, so daß der lipophile Tensidrest nicht mehr in das hydrophobe Enzyminnere eindringen kann. Diese Variante der Erfindung eignet sich insbesondere zum Abschirmen von hydrophoben Löchern gegen die destabilisierende Einwirkung eines lipophilen Tensidrestes. Ein Beispiel für Variante (b) der Erfindung mit einem hydrophoben Bereich, bei dem der Austausch einer angrenzenden polaren ungeladenen Aminosäure durch eine sterisch anspruchsvollere Aminosäure zu einer stabilisierten hochalkalischen Protease führt, ist in Fig. 10 dargestellt. Dieser hydrophobe Bereich ist als ein tief in das hydrophobe Enzyminnere eindringendes Loch ausgebildet, welches von einem polaren, durch die Aminosäuren His 118, Lys 229, Asn 237 und Arg 143 gebildeten Bereich umgeben ist. Die polaren ungeladenen Aminosäuren His 118 und Asn 237 grenzen direkt an den hydrophoben Bereich an. Der Ersatz der Aminosäure His 118 oder der Aminosäure Asn 237 durch eine der hier bspw. sterisch anspruchsvolleren Aminosäuren Tryptophan, Tyrosin oder Prolin führt zu erfindungsgemäß stabilisierten hochalkalischen Proteasen mit z.B. einer der Mutationen H118W, H118Y oder N237P.

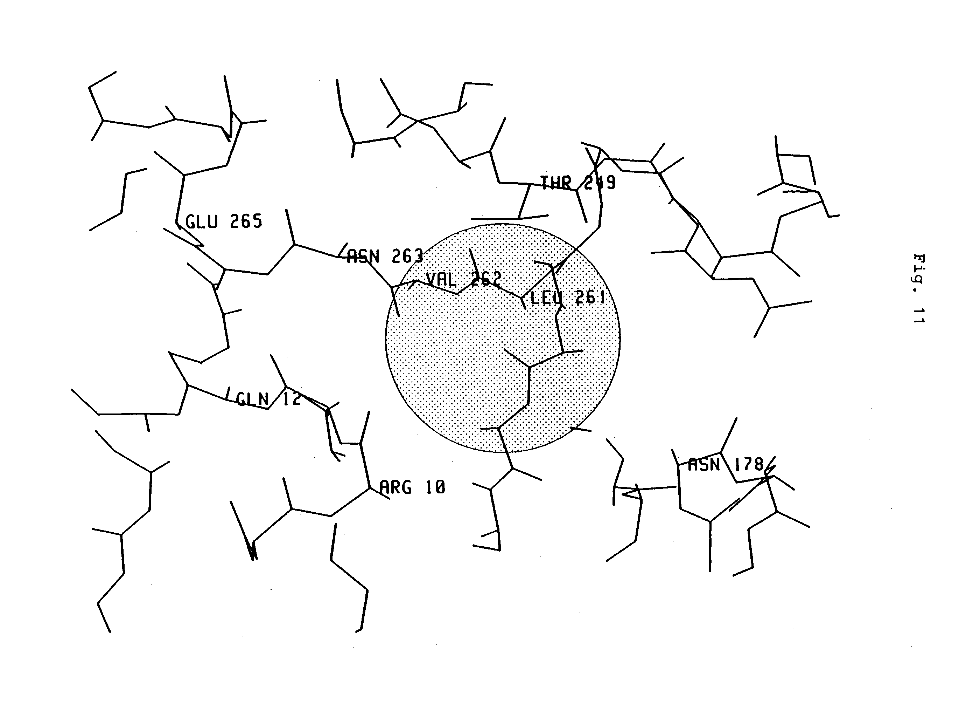

Vorzugsweise wird der apolare Bereich der Enzymoberfläche in Variante (b) der Erfindung durch eine gegenüber der ursprünglichen Aminosäure sterisch anspruchsvollere Aminosäure mit einem langkettigeren hydrophilen Aminosäurerest abgeschirmt. Ein Beispiel für einen solchen hydrophoben Bereich, bei dem der Austausch einer angrenzenden polaren ungeladenen Aminosäure durch eine langkettigere hydrophile Aminosäure zu einer stabilisierten hochalkalischen Protease führt, ist in Fig. 11 dargestellt. Dieser hydrophobe Bereich wird von den Aminosäuren Leu 261 und Val 262 gebildet und ist von einem polaren Bereich aus den Aminosäuren Glu 265, Asn 263, Gln 12, Arg 10, Asn 178 umgeben. Die polare ungeladene Aminosäure, die an den hydrophoben Bereich bei Leu261-Val262 angrenzt, ist Thr 249. Der Ersatz der Aminosäure Thr 249 durch bspw. eine der langkettigeren hydrophilen Aminosäuren Arginin, Lysin, Glutamin oder Glutaminsäure führt zu erfindungsgemäß stabilisierten hochalkalischen Proteasen mit z.B. einer der Mutationen T249R, T249K, T249Q, und T249E.

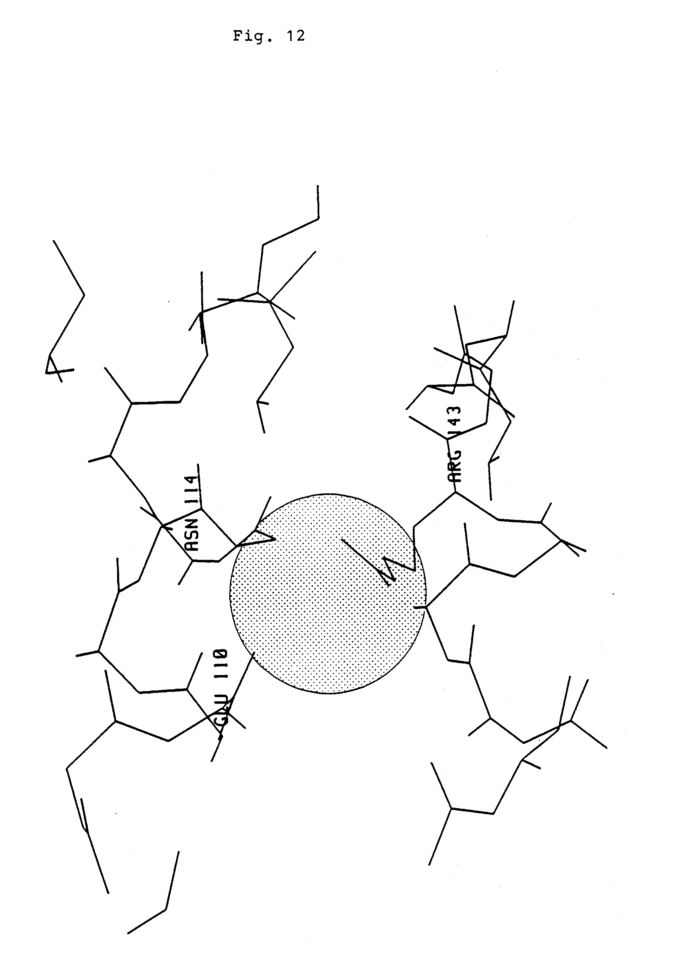

Gemäß Variante (c) der Erfindung ist anstelle einer ionischen Aminosäure, die in Nachbarschaft zu einem apolaren Bereich der Enzymoberfläche angesiedelt ist und deren geladener Aminosäurerest einen Anknüpfungspunkt für das ionische Tensid bilden kann, eine ungeladene hydrophile oder eine zum Tensid gleichgerichtet geladene Aminosäure eingeführt. Die auszutauschenden ionischen als auch die zum Tensid gleichgerichtet geladenen Aminosäuren im Sinne der Erfindung sind hierbei die oben bereits genannten hydrophilen Aminosäuren mit geladenen Aminosäureresten, d.h. Asparaginsäure, Glutaminsäure, Lysin und Arginin. In Enzymen, die gegen kationische Tenside stabilisiert sind, ist eine der anionischen Aminosäuren wie Asparaginsäure und Glutaminsäure gegen eine kationische Aminosäure wie Lysin oder Arginin ausgetauscht. Demgegenüber ist in Enzymen, die gegen anionische Tenside stabilisiert sind, gemäß dieser Variante der Erfindung eine kationische Aminosäure wie Lysin und Arginin gegen eine anionische Aminosäure ausgetauscht. In einer weiteren, hier bevorzugten Ausgestaltung der Variante (c) der Erfindung ist die auszutauschende ionische Aminosäure jedoch durch eine ungeladene hydrophile Aminosäure ausgetauscht. Ungeladene hydrophile Aminosäuren für diese bevorzugte Ausgestaltung sind insbesondere die Aminosäuren Serin (= Ser, S), Threonin (Thr, T), Asparagin (= Asp, N), Glutamin (= Gln, Q), Tyrosin (= Tyr, Y); die Aminosäuren mit kürzeren polaren Aminosäureresten wie Serin, Threonin und insbesondere Asparagin sind bevorzugt, in den Fällen, in denen die Ladung der ursprünglichen ionischen Aminosäure vom Enzym weit in das umgebende Medium hineinragt. Die Einführung einer ungeladenen hydrophilen Aminosäure für die ursprüngliche, ionische Aminosäure führt zu Enzymen, die sowohl gegen die Einwirkung von kationischen als auch von anionischen Tensiden stabilisiert sind. Ein Beispiel für einen hydrophoben Bereich der Enzymoberfläche, in dem ein Aminosäureaustausch gemäß Variante (c) der Erfindung zu einer stabilisierten hochalkalischen Protease aus Bacillus alcalophilus führt, ist in Fig. 12 dargestellt. Dieser hydrophobe Bereich ist als ein tief in das hydrophobe Enzyminnere eindringendes Loch ausgebildet und ist von den Aminosäuren Glu 110, Asn 114 und Arg 143 umgeben. Der Austausch der ionischen Aminosäure Arg 143, die einen bevorzugten Anknüpfungspunkt für ein anionisches Tensid bilden kann, durch bspw. insbesondere Asparagin, Serin oder Threonin führt zu erfindungsgemäß stabilisierten hochalkalischen Proteasen mit z.B. einer der Mutationen R143N, R143S oder R143T.

Die oben beschriebenen, erfindungsgemäß stabilisierten hochalkalischen Proteasen sind bspw. solche, die durch Kultivierung von Mikroorganismen wie z.B. Bacillus alcalophilus erhältlich sind. Insbesondere sind diese hochalkalischen Bacillus-Proteasen durch Kultivierung von Bacillus-Spezies erhältlich, die die Identifizierungsmerkmale von Bacillus alcalophilus DSM 5466 aufweisen. Die erfindungsgemäß stabilisierten Proteasen besitzen somit eine durch die oben angegebene Homologie definierte Verwandtschaft zu der aus Bacillus alcalophilus DSM 5466 erhältlichen Protease, deren Aminosäurensequenz in Fig. 1 als Bezugspunkt für die erfindungsgemäß stabilisierten Proteasen angegeben ist. Diese hochalkalischen Proteasen besitzen Molekulargewichte von 26000 bis 28000 g/mol, gemessen durch SDS-Polyacrylamid-Gelelektrophorese gegenüber Referenzproteinen mit bekanntem Molekulargewicht. Sie zeichnen sich weiterhin durch ein pH-Optimum im Bereich von 10 bis 12,5 aus, wobei unter pH-Optimum derjenige pH-Bereich verstanden wird, in dem die Proteasen maximale proteolytische Aktivität aufweisen und die Proteasen eine gute pH-Stabilität besitzen.

Die oben beschriebenen, erfindungsgemäß stabilisierten Enzyme können durch das in den Ansprüchen angegebene Verfahren zur Stabilisierung hergestellt werden. Dieses Verfahren zur Verbesserung der Stabilität von Enzymen gegen destabilisierende Einwirkungen von ionischen Tensiden zeichnet sich dadurch aus, daß man wengistens eine der Aminosäuren, die sich in einem hydrophoben Oberflächenbereich des Enzyms oder in direkter Nachbarschaft zu diesem hydrophoben Oberflächenbereich befinden, durch eine andere Aminosäure austauscht, wobei

(a) eine hydrophobe Aminosäure, deren Aminosäurerest an der Bildung eines hydrophoben Oberflächenbereiches beteiligt ist, in dessen räumlicher Nachbarschaft sich wenigstens eine Aminosäure mit ionischem Aminosäurerest befindet, durch eine andere Aminosäure mit hydrophilem Aminosäurerest ausgetauscht wird, und/oder wobei

(b) eine polare, ungeladenen Aminosäure, die an einem hydrophoben Oberflächenbereich angrenzt, in dessen räumlicher Nachbarschaft sich wenigstens eine Aminosäure mit ionischem Aminosäurerest befindet, durch eine gegenüber der ursprünglichen Aminosäure sterisch anspruchvollere Aminosäure, vorzugsweise durch eine sterisch anspruchvollere Aminosäure mit einem langkettigen hydrophilen Aminosäurerest, ausgetauscht wird, und/oder wobei

(c) eine ionische Aminosäure, die sich in räumlicher Nachbarschaft zu einem hydrophoben Oberflächenbereich befindet, durch eine Aminosäure mit einen ungeladenen hydrophilen Aminosäurerest oder durch eine geladene Aminosäure, deren Aminosäurerest eine zum ionischen Tensid gleichgerichtete Ladung aufweist, ausgetauscht wird.

Nach dem erfindungsgemäßen Verfahren werden in der Aminosäurensequenz des jeweils zu stabilierenden Enzyms gemäß den Vorgaben der Vefahrensvarianten (a), (b) und/oder (c) Aminosäuren durch gerichtete Mutagenese der DNA-Sequenz, die für die Aminosäurensequenz des Enzyms codiert, ausgetauscht. Die nachfolgende Expression der mutierten DNA-Sequenz mit Hilfe eines geeigneten Mikroorganismus liefert dann das durch Aminosäurenaustausch erfindungsgemäß stabilisierte Enzym. Hierbei kann zur Durchführung des Verfahrens im Einzelnen so vorgegangen werden, daß man

a) zunächst aus einem geeigneten Mikroorganismus, welcher das zu stabilisierende Enzym produziert, die für das Enzym codierende DNA-Sequenz (d.h. das Strukturgen des Enzyms) isoliert;

b) die Nukleotidabfolge dieser DNA-Sequenz bestimmt;

c) in der nunmehr bekannten DNA-Sequenz solche Mutationen erzeugt, daß die mutierte DNA-Sequenz nun für ein Enzym codiert, in dem eine Aminosäure des Ursprungsenzyms gemäß den Vorgaben der Verfahrensvarianten (a), (b) und/oder (c) durch eine andere Aminosäure ausgetauscht ist;

d) nachfolgend die mutierte DNA-Sequenz in einen geeigneten Expressionsvektor einbaut;

e) mit dem erhaltenen Expressionsvektor einen geeigneten Mikroorganismus, welcher schließlich zur Herstellung des mutierten Enzyms eingesetzt werden kann, transformiert.

Die einzelnen Verfahrensschritte zur erfindungsgemäßen Stabilisierung und zur Gewinnung der erfindungsgemäß stabilisierten Enzyme, sowie die hierbei erhaltenen Produkte sowie Zwischenprodukte in Form von DNA-Sequenzen, Vektoren, insbesondere Expressionsvektoren, und transformierten Mikroorganismen werden nachfolgend am Beispiel der hochalkalischen Proteasen näher beschrieben. Zur erfindungsgemäßen Stabilisierung anderer Enzyme - bspw. Lipasen, Amylasen, Cellulasen etc. - kann in analoger Weise vorgegangen werden.

Zu Herstellung von erfindungsgemäß stabilisierten hochalkalischen Proteasen wird zunächst aus einem geeigneten Bakterium, welches eine hochalkalische Protease mit einer Aminosäurensequenz mit mindestens 80 %, vorzugsweise mindestens 90 %, insbesondere aber mindestens 95 % Homologie zu der Aminosäurensequenz der Fig. 1 produziert, die für die Protease codierende DNA-Sequenz (d.h. das Strukturgen der Protease) isoliert. Die Strukturgene, die für Aminosäurensequenzen dieser hochalkalischen Proteasen codieren, können nach an sich bekannten, allgemeinen Methoden erhalten werden. Hierzu wird z.B. aus einem Bakterium ("Donor-Bakterium"), insbesondere aus einer Bacillus-Spezies, die die hochalkalische Protease produziert, die chromosomale DNA nach an sich bekannten Methoden isoliert und mit geeigneten Restriktionsendonukleasen in ansich bekannter Weise partiell hydrolysiert. Die hierdurch erhaltenen Restriktionsfragmente der Donor-DNA können z.B. durch Gelelektrophorese nach Größe aufgetrennt und die Fragmente gewünschter Größe dann mit einer geeigneten, doppelsträngigen Vektor-DNA in vitro verbunden (ligiert) werden (Rekombination). Häufig verwendete Vektoren sind die sogenannten Plasmide, d.h. extrachromosomale, ringförmige, doppelsträngige Bakterien-DNA, die sich durch geeignete Methoden (Transformation) in Mikroorganismen einbringen läßt und dort vermehrbar (autonom replizierbar) ist. Gegebenenfalls besitzen die Plasmide sogenannte Marker, d.h. DNA-Fragmente, die für bestimmte, beobachtbare Eigenschaften (z.B. Antibiotika-Resistenz) codieren und die zur Selektion der transformierten Mikroorganismen (Transformanten) dienen können.

Mit den vorstehenden Plasmiden (aus Vektor-DNA + Restriktionsfragmenten der Donor-DNA) können Bakterien, vorzugsweise eine Bacillus-Spezies, transformiert werden und die Transformanten nach der bekannten Markereigenschaft (z.B. Neomycin-Resistenz) selektiert werden. Man erhält so Klone, d.h. genetisch identische Transformanten. Unter diesen Transformanten können solche, die vermehrt Protease ausscheiden, auf proteinhaltigen Platten gesucht und danach isoliert werden. Aus einem Klon mit Proteaseaktivität wird schließlich die in diesen Transformanten eingeführte Plasmid-DNA isoliert und durch erneute Transformation eines Bakteriums überprüft, ob die Proteaseaktivität Plasmid-gebunden ist, d.h. ob die Proteaseaktivität mit der Markereigenschaft gekoppelt ist.

Das so isolierte Plasmid enthält neben der Vektor-DNA mit bekannten Restriktionsstellen das gewünschte Strukturgen für die zu stabilisierende hochalkalische Ausgansprotease und weitere, hier aber nicht benötigte DNA-Sequenzen aus dem Donor-Bakterium. Um den Aufwand für die nachfolgende Sequenzierung des Strukturgens der zu stabilisierenden hochalkalischen Protease möglichst gering zu halten, empfiehlt es sich, vor der eigentlichen Sequenzierung die zusätzlichen, nicht benötigten DNA-Sequenzen aus der Donor-DNA-Sequenz zu eliminieren und die Donor-DNA-Sequenz im wesentlichen auf das Strukturgen für die Protease zu reduzieren. Hierzu wird z.B. das Plasmid, welches das Strukturgen und die zusätzliche DNA-Sequenz umfaßt, mit einer Anzahl verschiedener Restriktionsendonukleasen geschnitten (restringiert), die erhaltenen DNA-Fragmente durch Gelelektrophorese nach Größe getrennt und anhand des gefundenen Bandenmusters eine Restriktionskarte erstellt. Es werden so die Restriktionsstellen, die im Bereich der Donor-DNA-Sequenz angesiedelt sind, ermittelt. Die Kenntnis der Restriktionskarte des Plasmids ermöglicht es, durch Schneiden mit ausgewählten Restriktionsendonukleasen ein DNA-Fragment aus der Donor-DNA-Sequenz herauszuschneiden, welches im wesentlichen nur das Strukturgen für die hochalkalische Protease, die zugehörigen Pre- und Pro-Einheiten, sowie die für die Genexpression benötigte Promotor-Einheit umfaßt.

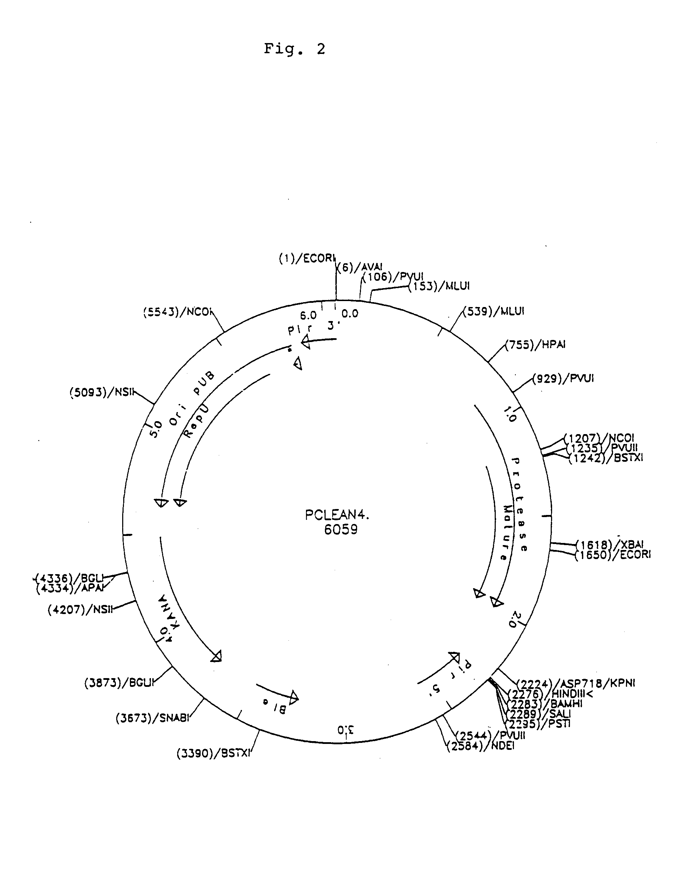

Durch den Wiedereinbau dieser in der Größe reduzierten Donor-DNA-Sequenz in einen geeigneten Vektor kann ein neuer, replizierbarer Vektor erhalten werden, dessen Fähigkeit zur Expression der hochalkalischen Ausgangsprotease überprüft werden kann, indem man ein Bakterium, insbesondere eine Bacillus-Spezies, mit diesem Vektor transformiert, den erhaltenen Transformanten kultiviert und auf Proteaseaktivität überprüft. Ein Beispiel für einen solchen reduzierten Vektor mit der Bezeichnung pCLEAN4 gibt die Restriktionskarte der Fig. 2 wieder.

Zur Bestimmung der Nukleotidsequenz (Sequenzierung) des Proteasestrukturgens wird zunächst der

6

vorstehend beschriebene Vektor in einem geeigneten Mikroorganismus repliziert, und das Proteasegen isoliert. Dieses wird sodann in einen Phagemiden subkloniert und die erhaltenen Phagemiden anschließend in einen geeigneten Mikroorganismus z.B. E. coli transformiert und durch Kultivierung der Transformanten einzelsträngige, das Proteasegen enthaltende DNA produziert. Die gebildete einzelsträngige DNA wird isoliert und der Sequenzierung zugeführt. Die Sequenzierung wird nach an sich bekannten Methoden ausgeführt, indem man z.B. die Einzelstrang-DNA mit dem Proteasegen nach Maxam und Gilbert einer basenspezifischen partiellen chemischen Spaltung zuführt (1980, in Methods in Enzymology, Grossmann L., Moldave K., eds., Academic Press Inc., New York und London, Vol. 65, 499), oder indem man z.B. die Einzelstrang-DNA mit dem Proteasegen als Matritze für die partielle Synthese von Teilstücken des komplementären DNA-Stranges nach der Dideoxy-Kettenterminator-Methode nach Sanger et al. (1977, Proc. Natl. Acad. Sci. USA 74: 5463) einsetzt.

Die ermittelte Nukleotidsequenz kann nunmehr mit Hilfe des genetischen Codes (ein Triplett-Wort = Codon steht für eine definierte Aminosäure) in die Aminosäurensequenz der Protease übersetzt werden. Zur Bestimmung des Anfangspunktes der Aminosäurensequenz des reifen Protease-Enzyms (d.h. das Enzym ohne die Pre- und Pro-Einheiten) wird am N-terminalen Ende der reifen Protease ein kurzes Stück der Aminosäurenabfolge durch an sich bekannte Methoden zur Bestimmung von Aminosäurensequenzen in Peptiden bestimmt. Die bekannte N-terminale Aminosäurensequenz kann nun anhand des genetischen Codes dem entsprechenden Teilstück der obigen Nukleotidsequenz zugeordnet werden und so der Anfangspunkt der für die reife Protease codierenden DNA-Sequenz festgelegt werden. Die weitere Aminosäurenabfolge der Protease ergibt sich dann zwangsläufig aus der DNA-Sequenz durch Zuordnung der nachfolgenden Aminosäuren mit Hilfe des genetischen Codes.

Erfindungsgemäß wird die für die hochalkalische Protease codierende DNA-Sequenz durch Änderung der entsprechenden Codons derart mutiert, daß die mutierte DNA-Sequenz für eine gegen Einwirkung von ionischen Tensiden stabilisierte hochalkalische Protease codiert, in deren Aminosäurensequenz in einer der Positionen 4, 14, 27, 39, 43, 47, 49, 77, 80, 89, 111, 117, 118, 127, 143, 161, 164, 165, 208, 232, 233, 235, 237, 249, 256 der Fig. 1 oder in einer der dazu homologen Positionen die betreffende Aminosäure durch eine Aminosäure gemäß einer der Verfahrensvarianten (a), (b) und/oder (c) ausgetauscht ist.

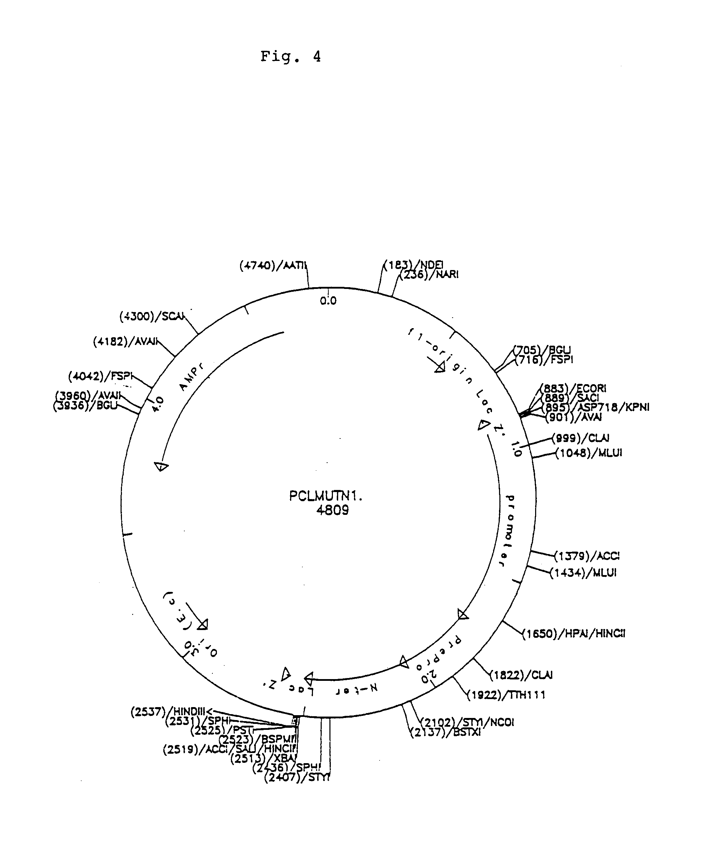

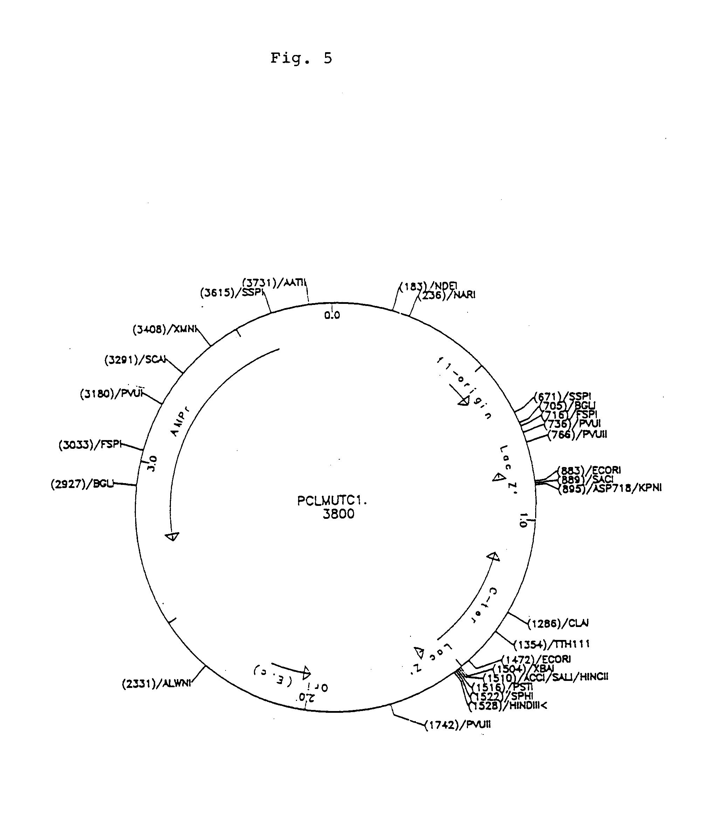

Die Einführung der Mutationen in die für die hochalkalische Protease codierende DNA wird durch an sich bekannte Methoden zur gerichteten Mutagenese bewerkstelligt. Hierzu wird aus geeigneten Vektoren (Phagemide), z.B. aus pCLMUTN1 der Fig. 4 oder pCLMUTC1 der Fig. 5, gegebenenfalls unter Mithilfe eines Helfer-Phagen, ringförmige Einzelstrang-DNA erzeugt, die das gesamte Strukturgen, oder aber vorzugsweise nur denjenigen Teil (z.B. nur den N-terminalen Teil bzw. den C-terminalen Teil) des Strukturgens der Ursprungsprotease, in welchem die Mutation vorgenommen werden soll, enthält. Mit dieser ringförmigen Einzelstrang-DNA hybridisiert man ein synthetisches, hybridisierfähiges Oligonukleotid, welches in der gewünschten Mutationsstelle ein Basentriplett (Codon) enthält, welches statt für die originäre, auszutauschende Aminosäure erfindungsgemäß für eine neue Aminosäure, gemäß (a), (b) oder (c) codiert. Zusätzlich ist das Oligonukleotid gegenüber der zu hybridisierenden, originären Nukleotidsequenz noch derart durch einen oder einige wenige weitere Nukleotidbausteine abgewandelt, daß die Codierung der originären Aminosäurensequenz zwar im Rahmen der Degeneration des genetischen Codes erhalten bleibt, jedoch in der originären Protease-Nukleotidsequenz eine gegebenenfalls vorhandene Restriktionsstelle im synthetischen Oligonukleotid entfernt bzw. eine weitere Restriktionsstelle in das synthetische Oligonukleotid eingeführt wird. Die entfernte bzw. eingeführte Restriktionsstelle dient später mit Hilfe geeigneter Restriktionsendonukleasen zur Identifizierung der Mutanten-DNA-Sequenz gegenüber der Ausgangstyp-DNA-Sequenz. Die durch Hybridisierung erhaltene, partiell doppelsträngige DNA-Sequenz wird durch Zugabe der benötigten Nukleotide und unter Einwirkung von DNA-Polymerase und DNA-Ligase zum vollständigen Doppelstrang ergänzt. Die erzeugte ringförmige, doppelsträngige DNA-Sequenz wird nachfolgend als Vektor in einen geeigneten Mikroorganismus transformiert und nach ausreichender Replikation die mutierten DNA-Sequenzen über die entfernte bzw. zusätzlich eingeführte Restriktionsstelle im Oligonukleotidsequenzteil identifiziert und anschließend isoliert.

In einer Verfahrensvariante wird in der gerichteten Mutagenese uracylierte Einzelstrang-DNA als Matrize erzeugt und für die Hybridisierung mit den synthetischen Oligonukleotiden verwendet. Nach Beendigung der Reaktionen des Verfahrens der gerichteten Mutagenese kann der Uracilhaltige DNA-Einzelstrang, der als Matrize zur Erzeugung mutierter DNA-Stränge (Vektoren) diente, durch Behandlung mit Uracil-N-Glycosylase beseitigt werden, ohne daß es einer phänotypischen Selektion von Mutanten bedarf. Die Glycosylase-Behandlung kann z.B. mit Hilfe eines geeigneten Mikroorganismus mit Uracil-N-Glycosylase-Aktivität durchgeführt werden, der mit mutierter Vektor-DNA transformiert wurde. Die Replikation kann z.B. in einem E. coli-Stamm vorgenommen werden, der nur den mutierten, nicht-uracylierten DNA-Strang des im Mutationsverfahren erzeugten Doppelstrang-Vektors vermehrt. Hierdurch wird die Selektion der mutierten DNA-Vektoren zusätzlich erleichtert.

Die für die gerichtete Mutagenese benötigten synthetischen Oligonukleotide werden nach an sich bekannten Methoden hergestellt. Beispielsweise kann die Herstellung der Oligonukleotide nach Beaucage S.L. und Caruthers M.H. (1981, Tetrahedron Letters 22: 1859 - 1862) mit $\beta$-Cyanoethylphosphoramidit in einem Cyclone-Synthetiser (Biosearch) erfolgen. Die erhaltenen Oligonukleotide können z.B. durch Elution aus Polyacrylamid-Gelen und gegebenenfalls anschließende Entsalzung mit Hilfe von Sephadex-Säulen gereinigt und der weiteren Verwendung zugeführt werden. Die synthetischen Oligonukleotide können direkt als Primer für die DNA-Polymerase im vorstehend beschriebenen Mutagenese-Verfahren dienen. Die synthetischen Oligonukleotidsequenzen umfassen z.B. 20 bis 30 Nukleotidbausteine, die für etwa 7 bis 10 Aminosäuren codieren. Es ist natürlich auch möglich längere Nukleotidsequenzen für die obige Hybridisierung einzusetzen, doch führt dies zu keinen weiteren Vorteilen, solange eine ausreichende Hybridisierungsfähigkeit der kurzkettigen synthetischen Oligonukleotide sichergestellt ist.

Die durch das oben beschriebene Verfahren der gerichteten Mutagenese erhaltenen ringförmigen, doppelsträngigen DNA-Sequenzen mit den eingeführten Mutationen stellen mutierte Vektoren dar, aus denen durch Behandlung mit geeigneten Restriktionsendonukleasen, je nach Fall, das gesamte mutierte Protease-Strukturgen oder das mutierte Teilstück des Protease-Strukturgens herausgeschnitten und in einen geeigneten Expressionsvektor eingebracht (subkloniert) werden kann. Mit diesem Expressionsvektor werden dann geeignete Mikroorganismen, z.B. Bacillus-Spezies, transformiert, die nachfolgend zur Expression und Gewinnung der mutierten hochalkalischen Proteasen unter geeigneten Bedingungen kultiviert werden.

In einer bevorzugten Ausgestaltung des Verfahrens wird nicht das gesamte Strukturgen für die gerichtete Mutagenese eingesetzt, sondern nur ein Teilstück desselben, in dem die Mutation erzeugt werden soll. Hierzu wird aus dem Vektor der zur Replikation der Strukturgene dient, z.B. die N-terminale oder C-terminale Hälfte des Strukturgens mit geeigneten Restriktionsendonukleasen herausgeschnitten und in einen passenden Phagemiden subkloniert. Man erhält so Vektoren, die entweder die N-terminale oder die C-terminale Hälfte des Strukturgens der Protease enthalten und die in einem geeigneten Mikroorganismus, z.B. E. coli, zunächst ausreichend repliziert und dann der oben beschriebenen, gerichteten Mutagenese zugeführt werden. Die Mutagenese von Teilstücken des Strukturgenes hat den Vorteil, daß kürzere Einzelstrang-DNA-Sequenzen verwendet werden können und somit nach dem Hybridisierungsschritt mit synthetischen Oligonukleotiden im partiellen DNA-Doppelstrang wesentlich weniger Nukleotide als bei Verwendung der gesamten DNA-Sequenz zu ergänzen sind. Hier durch wird der synthetische Aufwand und zusätzlich die Gefahr unerwünschter zufälliger Mutationen reduziert.

Die mutierten DNA-Sequenzen können aus dem zur Erzeugung der Mutationen dienenden Klonierungsvektoren durch geeignete Restriktionsendonukleasen herausgeschnitten und in Vektoren mit passenden Restriktionsstellen eingebaut werden. Die erhaltenen Vektoren sind bspw. Vorläufer der eigentlichen, für die Expression der hochalkalischen Protease benötigten Expressionsvektoren. Diese Vektoren sind so aufgebaut, daß sie außer den geeigneten Restriktionsstellen (z.B. aus einem synthetischen Linker) auch bereits die für die Proteaseexpression in einem Wirtsorganismus benötigten regulatorischen Sequenzen, Signalsequenzen, Promotorsequenzen und die für die Pre- und Proeinheiten der Protease codierenden DNA-Sequenzen enthalten.

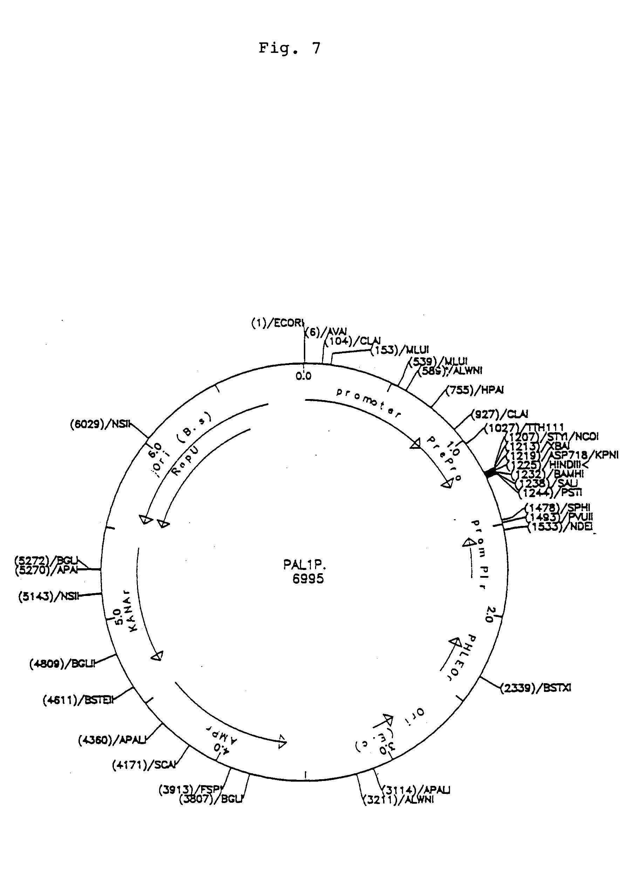

Durch die Subklonierung einer mutierten DNA-Sequenz in einen solchen Vektor wird der eigentliche Expressionsvektor für eine optimierte hochalkalische Protease erhalten. Der Einbau der mutierten DNA-Sequenz in diesen Vorläufer des Expressionsvektors erfolgt so, daß ein Expressionsvektor mit geeignetem Leseraster entsteht. Hierbei können mutierte Teilstücke der für die Protease codierenden DNA-Sequenz, z.B. ein C-terminales oder ein N-terminales Teilstück, in bereits das jeweils restliche nicht mutierte Teilstück enthaltende Vektoren eingebaut werden; oder es wird die gesamte für die Protease codierende mutierte DNA-Sequenz in Vektoren, welche noch keine Teilstücke dieser Protease-DNA-Sequenz enthalten, eingebaut. Beispiele für solche, bereits ein Teilstück der nicht-mutierten DNA-Sequenz enthaltende Vorläufervektoren eines Expressionsvektors sind die weiter unten und in den Beispielen näher beschriebenen Vektoren mit der Bezeichnung pAL1N und pAL1C. Ein Vektor, der noch kein Teilstück der Protease-DNA-Sequenz enthält, ist der Vektor pAL1P mit der in Fig. 7 angegebenen Restriktionskarte.

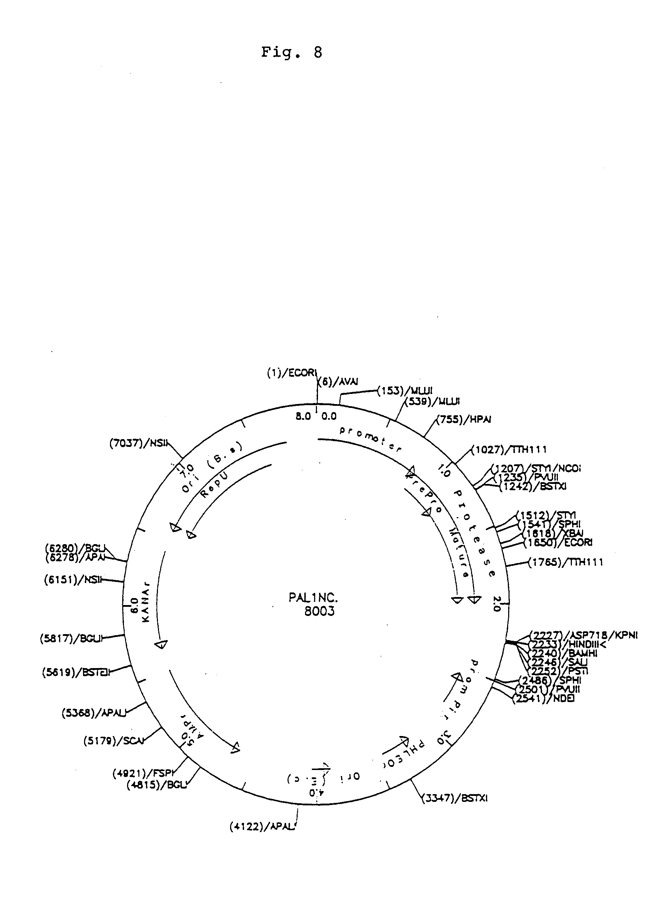

Die Expressionsvektoren-Vorläufer für die bevorzugte Variante der Erfindung (Mutation in der N-terminalen Hälfte oder in der C-terminalen Hälfte) werden z.B. wie folgt erhalten. Zunächst führt man in ein Bacillus-Plasmid eine Polyklonierungsstelle ein. Das so erhaltene Plasmid wird restringiert und mit einem E. coli-Plasmidfragment, welches Marker und für die Replikation wichtige Sequenzteile enthält, rekombiniert. Anschließend werden gegebenenfalls solche Restriktionsstellen z.B. durch gerichtete Mutagenese entfernt, die spätere Verfahrensschritte stören würden. Aus dem so erhaltenen Plasmid wird ein neuer Vektor konstruiert, der die aus dem Bacillus-Plasmid und dem E. coli-Plasmid zur Replikation dienenden DNA-Sequenzen, DNA-Sequenzen für den Promotor, DNA-Sequenzen, die für die Pre-Prosequenz der Protease codieren (erhalten aus z.B. dem Plasmid pCLEAN4 der Fig. 2), und einen synthetischen Linker enthält. Ein Beispiel für ein solches Plasmid mit der Bezeichnung pAL1P gibt die Restriktionskarte der Fig. 7 wieder. Der synthetische Linker ist dabei derart ausgewählt, daß nach Schneiden mit geeigneten Restriktionsendonukleasen entweder mit dem gesamten Ursprungsstrukturgen bzw. dem gesamten mutierten Strukturgen bzw. mit mutierten oder nicht mutierten Teilstücken des Strukturgens rekombiniert werden kann. Zur Herstellung eines Expressionsvektor-Vorläufers, der z.B. mit einer mutierten N-terminalen Hälfte des Strukturgens rekombiniert werden soll, wird in den vorstehend konstruierten Vektor, der die genannten Bacillus-, E. coli-, die Promotor- und die Pre- und Prosequenzen der Protease sowie den synthetischen Linker enthält, durch Schneiden des synthetischen Linkers z.B. zunächst die nichtmutierte C-terminale Hälfte des Strukturgens der Protease eingeführt. Man erhält so die bereits genannten Vektoren vom Typ pAL1C. Anschließend wird durch nochmaliges Schneiden des synthetischen Linkers die noch fehlende, mutierte N-terminale Hälfte des Protease-Strukturgens eingeführt. Auf diese Weise erhält man einen Vektor vom Typ pAL1NC der Fig. 8. Analoges gilt für den umgekehrten Fall. Es wird dann zunächst die nicht-mutierte N-terminale Hälfte in einen Vektor vom Typ pAL1P und in den so erhaltenen Vektor vom Typ pAL1N später die mutierte C-terminale Hälfte eingeführt, wobei man ebenfalls einen Vektor vom Typ pAL1NC der Fig. 8 erhält.

Mit den oben beschriebenen Expressionsvektoren werden geeignete Bakterien, vorzugsweise Bacillus-Spezies, insbesondere Bacillus subtilis, licheniformis und alcalophilus, transformiert. Die Transformanten werden anschließend in an sich bekannter Weise kultiviert und die gebildete, erfindungsgemäß stabilisierte hochalkalische Protease aus dem Kulturmedium isoliert. Die Expressionsvektoren können hierzu sowohl in Bakterien, die noch zur Bildung von Eigenprotease befähigt sind, als auch in Protease-defiziente Bakterien (die keine Eigenprotease mehr bilden) transformiert werden. Bei Wirtsorganismen mit Eigenproteasebildung kann die erfindungsgemäß stabilisierte hochalkalische Protease gewünschtenfalls durch anschließende Reinigungsoperationen, z.B. durch hochauflösende Flüssigchromatographie (HPLC), von den gebildeten Eigenproteasen befreit werden. Ein solcher Reinigungschritt kann demgegenüber bei Protease-defizienten Wirtsorganismen entfallen, da diese nur (oder im wesentlichen nur) die stabilisierte Protease zu bilden vermögen.

Durch das erfindungsgemäße Verfahren zur Stabilisierung von Enzymen gegen destabilierende Einwirkungen von ionischen Tensiden werden auch neue DNA-Sequenzen erzeugt, die ebenfalls einen Erfindungsgegenstand darstellen. Diese erfindungsgemäßen DNA-Sequenzen zeichnen sich dadurch aus, daß sie für eine hochalkalische Protease codieren, deren Aminosäurensequenz mindestens 80 %, vorzugsweise mindestens 90 %, insbesondere aber mindestens 95 % Homologie zu der in Fig. 1 angegebenen Aminosäurensequenz aufweist und sich von dieser in einer der Positionen 4, 14, 27, 39, 43, 47, 49, 77, 80, 89, 111, 117, 118, 127, 143, 161, 164, 165, 208, 232, 233, 235, 237, 249 oder 256, vorzugsweise 27, 43, 118, 143, 164, 237 oder 249, der Fig 1 oder in einer der dazu homologen Positionen, die sich in einem hydrophoben Oberflächenbereich der Protease oder in Nachbarschaft zu diesem hydrophoben Oberflächenbereich befinden, dadurch unterscheidet, daß die in der betreffenden Position befindliche Aminosäure im Falle von Positionen mit

(a) einer hydrophoben Aminosäure, deren Aminosäurerest an der Bildung eines hydrophoben Oberflächenbereiches beteiligt ist, in dessen räumlicher Nachbarschaft sich wenigstens eine Aminosäure mit ionischem Aminosäurerest befindet, durch eine andere Aminosäure mit hydrophilem Aminosäurerest ersetzt ist, und/oder daß im Falle von Positionen mit

(b) einer polaren ungeladenen Aminosäure, die an einen hydrophoben Oberflächenbereich angrenzt, in dessen räumlicher Nachbarschaft sich wenigstens eine Aminosäure mit ionischem Aminosäurerest befindet, durch eine gegenüber der ursprünglichen Aminosäure sterisch anspruchvollere Aminosäure, vorzugsweise durch eine sterisch anspruchsvollere Aminosäure mit einem langkettigeren, hydrophilen Aminosäurerest, ersetzt ist, und/oder daß im Falle von Positionen mit

(c) einer ionischen Aminosäure, die sich in räumlicher Nachbarschaft zu einem hydrophoben Oberflächenbereich befindet, durch eine Aminosäure mit einem ungeladenen hydrophilen Aminosäurerest oder durch eine geladene Aminosäure, deren Aminosäurerest eine zum ionischen Tensid gleichgerichtete Ladung aufweist, ersetzt ist.

Positionen hydrophober Aminosäuren, die sich in einem hydrophoben Bereich der Enzymoberfläche befinden sind bspw. Val 4, Pro 14, Pro 39, Ile 43, Ala 47, Phe 49, Ile 77, Leu 80, Trp 111, Met 117, Pro 127, Pro 233, Trp 235, Leu 256. Positionen polarer ungeladener Aminosäuren, die an einen hydrophoben Bereich der Enzymoberfläche angrenzen sind bspw. die Tyrosinpositionen 89, 161, 165, 208; die Asparaginpositionen 232 und 237; die Histidinposition 118 und die Threoninposition 249. Beispiele für Positionen ionischer Aminosäuren, die sich in Nachbarschaft zu einem hydrophoben Bereich befinden, sind insbesondere die Argininpositionen 143 und 164 sowie die Lysinposition 27.

Die erfindungsgemäß stabilisierten Enzyme eignen sich einzeln oder in Kombination miteinander oder mit anderen im Stand der Technik bekannten Enzymen hervorragend für die Anwendung in festen und flüssigen Wasch- und Reinigungsmittelformulierungen, insbesondere aber für die Anwendung in Flüssigformulierungen. Diese Wasch- und Reinigungsmittelformulierungen können in an sich üblicher Weise formuliert werden. Die erfindungsgemäß stabilisierten Enzyme können dazu z.B. in Form von Granulaten, Prills oder Pellets, gegebenenfalls auch mit Oberflächenüberzügen versehen, oder bei Flüssigformulierungen auch in gelöster Form, mit den anderen Komponenten der Wasch- und Reinigungsmittelformulierung in an sich bekannter Weise vermischt werden. Die erfindungsgemäß stabilisierten Enzyme können in den Formulierungen in für Wasch- und Reinigungsmittelenzyme üblichen Mengen, insbesondere in Mengen von bis zu 3 Gew.-% (bezogen auf die Trockensubstanz der Gesamtzusammensetzung), vorzugsweise in einer Menge von 0,2 bis 1,5 Gew.-%, eingesetzt werden. Die Erfindung umfaßt daher weiterhin Wasch- und Reinigungsmittelformulierungen, die wenigstens eines der erfindungsgemäß stabilisierten Enzyme enthalten; insbesondere solche Formulierungen, die erfindungsgemäße Enzyme und ionische, vorzugsweise anionische Tenside, enthalten. Außer den erfindungsgemäßen Enzymen können in den Wasch- und Reinigungsmittelformulierungen weiterhin auch an sich bekannte Enzyme und alle an sich im Stand der Technik üblichen Waschmittelinhaltsstoffe wie Tenside, Bleichmittel oder Gerüststoffe (Builder), sowie weitere übliche Hilfsstoffe für die Formulierung von Waschmitteln in an sich üblichen Mengen enthalten sein. Zu den Hilfsstoffen gehören z.B. Verstärker, Enzymstabilatoren, Schmutzträger und/oder Kompatibilisierungsmittel, Komplex- und Chelatbildner, Seifenschaumregulatoren und Zusatzstoffe wie optische Aufheller, Opazifizierungsmittel, Korrosionsinhibitoren, Antielektrostatika, Farbstoffe, Bakterizide, Bleichmittelaktivatoren, Persäurebleichmittelvorstufen. So enthalten erfindungsgemäße Wasch- und Reinigungsmittelformulierungen in typischer beispielhafter Zusammensetzung bezogen auf Trockensubstanz

a) wenigstens 5 Gew.-% z.B. 10 bis 50 Gew.-%, eines ionischen, vorzugsweise anionischen Tensids oder eines Gemisches dieser Tenside;

b) bis zu 40 Gew.-% eines Builders oder eines Builder-Gemisches;

c) bis zu 40 Gew.-% eines Bleichmittels oder Bleichmittelgemisches, vorzugsweise ein Perborat wie Natriumperborat-Tetrahydrat oder Natriumperborat-Monohydrat;

d) bis zu 3 Gew.-% wenigstens eines erfindungsgemäßen Enzyms, insbesondere einer erfindungsgemäßen Protease;

e) weitere Bestandteile wie Hilfsstoffe etc. zur Ergänzung auf 100 Gew.-%

Erläuterungen zu den Figuren:

Figur 1:
Sequenzprotokoll für die DNA-Sequenz des AvaI/HindIII-Fragmentes mit dem Strukturgen der hochalkalischen Ausgangsprotease aus Bacillus alcalophilus HA1, sowie die Aminosäurensequenz dieser Ausgangsprotease.

Figur 2:
Restriktionskarte des Plasmides pCLEAN4.

Figur 3:
Beispiele für DNA-Sequenzen für zur gerichteten Mutagenese verwendete synthetische Oligonukleotide und Angabe eliminierter bzw. erzeugter Erkennungsstellen für einzelne Restriktionsendonukleasen; die gegenüber der ursprünglichen DNA-Sequenz der Ausgangsprotease erzeugten Nukleotidveränderungen sind durch Angabe der veränderten Nukleotide mit kleinen Buchstaben gekennzeichnet.

Figur 4:
Restriktionskarte des Vektors pCLMUTN1.

Figur 5:
Restriktionskarte des Vektors pCLMUTC1.

Figur 6:
Restriktionskarte des Vektors pUBC132.

Figur 7:
Restriktionskarte des Plasmides pAL1P.

Figur 8:
Restriktionskarte der Expressionsvektoren vom Typ pAL1NC für die Expression der durch Mutation stabilisierten hochalkalischen Proteasen und der nicht-mutierten hochalkalischen Ausgangsprotease.

Figur 9:

Die Figur zeigt einen flachen hydrophoben Bereich bei der Position Ile 43.

Figur 10:

Die Figur zeigt ein hydrophobes, tief in das Enzyminnere eindringendes Loch, an das die austauschbaren, polaren ungeladenen Aminosäuren His 118 und Asn 237 angrenzen.

Figur 11:

Die Figur zeigt einen hydrophoben Bereich bei den Positionen Val261-Leu262.

Figur 12:

Die Figur zeigt ein hydrophobes, tief in das Enzyminnere eindringendes Loch, in dessen Nachbarschaft sich die austauschbare, ionische Aminosäure Arg 143 befindet.

Beispiele

Die folgende Offenbarung gibt zur weiteren Erläuterung der Erfindung typische beispielhafte Ausgestaltungen der Erfindung am Beispiel einer hochalkalischen Protease aus Bacillus alcalophilus HA1 wieder, ohne jedoch dadurch die Erfindung zu beschränken.

Um die Beispiele zu vereinfachen werden einige häufig wiederkehrende Methoden und Begriffe im folgenden näher erläutert und dann in den einzelnen Beispielen nur noch durch eine Kurzbezeichnung referiert. Sofern nicht anders angegeben, wurde generell nach Methoden gearbeitet, wie sie in Maniatis et al. (Maniatis et al. = T. Maniatis, E. F. Fritsch, J. Sambrook, Molecular Cloning, A Laboratory Manual, Cold Spring Harbor Laboratory, 1982) beschrieben sind.

Hier verwendete Ausgangsvektoren sind käuflich und auf unbeschränkter Basis verfügbar; oder sie können nach an sich bekannten Methoden aus verfügbaren Vektoren hergestellt werden. Beispielsweise wurden Phagemide mit der Bezeichnung pBS (z.B. pBS(+), pBS(-) etc.) von Stratagene, La Jolla, Kalifornien bezogen; der Helfer-Phage M13K07 von Bio-Rad Laboratories, Richmond, Kalifornien; der Vektor M13tg131 von Amersham, Buckinghamshire, England; das Plasmid pUC18 von Pharmacia LKB, Uppsala, Schweden.

Die verschiedenen benutzten Restriktionsendonukleasen gehören zum Stand der Technik und sind kommerziell verfügbar. Die bei Verwendung dieser bekannten Restriktionsendonukleasen jeweils erforderlichen Reaktions-, Kofaktor- und übrigen Bedinungen sind ebenfalls bekannt.

An das Schneiden von Vektoren mit Restriktionsendonukleasen kann sich gegebenenfalls eine Hydrolyse des terminalen 5'-Phosphatrestes mit einer alkalischen Phosphatase (Dephosphorylierung) anschließen. Dadurch kann verhindert werden, daß die beim Schneiden entstandenen Enden des restringierten Vektors mit sich selbst rekombinieren und somit die gewünschte Insertion eines Fremd-DNA-Fragmentes in die Restriktionsstelle verhindert würde. Sofern in den Beispielen eine Dephosphorylierung des 5'-Endes vorgenommen wurde, geschah dieses in an sich bekannter Weise. Weitere Angaben zur Durchführung einer Dephosphorylierung und zu dafür benötigten Reagentien können Maniatis et al. (S. 133 - 134) entnommen werden.

Partielle Hydrolyse bedeutet unvollständige Verdauung von DNA durch eine Restriktionsendonuklease. Die Reaktionsbedingungen werden dabei so gewählt, daß in einem DNA-Substrat zwar an einigen, nicht aber an allen Erkennungsstellen für die eingesetzte Restriktionsendonuklease geschnitten wird.

Zur Gewinnung und Isolierung von bestimmten DNA-Fragmenten, z.B. nach Behandlung von DNA mit Restriktionsendonukleasen, wurden die angefallenen DNA-Fragemente in an sich bekannter Weise durch Gelelektrophorese (z.B. auf Agarosegel) getrennt, nachfolgend über das Molekulargewicht (Bestimmung durch Vergleich mit Referenz-DNA-Fragmenten mit bekanntem Molekulargewicht) identifiziert und die gewünschten DNA-Fragmente aus den entsprechenden Gelzonen abgetrennt.

Behandlung mit dem Klenow-Fragment der DNA-Polymerase I aus E. coli bedeutet ein Verfahren zum Auffüllen der inneren 3'-Enden von doppelsträngiger DNA mit Nukleotiden, die zu den Nukleotiden der jeweiligen überstehenden 5'-Enden des DNA-Doppelstranges komplementär sind. Das Klenow-Fragment sowie für die Klenow-Behandlung benötigte Reagentien sind im Stand der Technik bekannt und kommerziell verfügbar. Details zur Klenow-Behandlung sind z.B. aus Maniatis et al. (S. 107 bis 108) entnehmbar.

Ligation (ligieren) bedeutet ein Verfahren zur Bildung von Phosphodiesterbindungen zwischen DNA-Fragmenten (siehe z.B. Maniatis et al., S. 146). Ligationen können unter an sich bekannten Bedingungen, z.B. in einem Puffer mit etwa 10 Units T4-DNA-Ligase pro 0,5 μg einer etwa gleich molaren Menge der zu ligierenden DNA-Fragmente, ausgeführt werden.

Unter Transformation wird die Einschleusung von DNA in einen Mikroorganismus verstanden, so daß die DNA in diesem repliziert bzw. exprimiert werden kann. Für die Transformation von E. coli ist z.B. die Calciumchloridmethode nach Mandel et al. (1970, J. Mol. Biol. 53: 159) oder nach Maniatis et al. (S. 250 bis 251) geeignet. Für Bacillus-Spezies ist z.B. die Methode Anagnostopoulos et al. (1961, J. Bact. 81: 741 - 746) geeignet.

Ein Linker ist ein kurzkettiges doppelsträngiges DNA-Fragment, welches einige Erkennungsstellen für Restriktionsendonukleasen aufweist und sich zum Verbinden von DNA-Fragmenten eignet. Linker werden z.B. beim Rekombinieren von DNA-Fragmenten zu einem Vektor eingesetzt und können zur Einführung bestimmter Erkennungsstellen für Restriktionsendonukleasen in diesen Vektor dienen.

Eine Polyklonierungsstelle (Polylinker) ist ein kurzes bis mittleres doppelsträngiges DNA-Fragment, welches eng benachbart eine Vielzahl von Erkennungsstellen für Restriktionsendonukleasen aufweist. Eine in den Beispielen verwendete, aus dem Vektor M13tg131 entstammende, Polyklonierungsstelle besitzt z.B. eine Größe von etwa 0,07 KB (Kilo Basenpaare) und weist Erkennungsstellen für 14 verschiedene Restriktionsendonukleasen auf.

Der im Beispiel 1 eingesetzte und als Bacillus alcalophilus HA1 benannte Bacillus alcalophilus-Stamm ist bei der Deutschen Sammlung von Mikroorganismen (DSM) mit der DSM-Nummer 5466 am 28. Juli 1989 hinterlegt worden. Andere verwendete Mikroorganismen sind käuflich verfügbar, z.B. Bacillus subtilis BD 244 (Bacillus Genetic Stock Center 1 A 46) oder Bacillus subtilis BD 366 (Bacillus Genetic Stock Center 1 E 6).

Beispiel 1

Herstellung einer genomischen DNA-Bibliothek und Isolierung des Gens für ein Enzym am Beispiel von B. alcalophilus und dessen hochalkalischer Protease

Aus dem Naturisolat Bacillus alcalophilus HA1 (hinterlegt bei der Deutschen Sammlung von Mikroorganismen unter der Nummer DSM 5466) wurde nach der Methode von Saito et al. (1963, Biochim.Biophys. Acta. 72:619-629) chromosomale DNA isoliert und mit der Restriktionsendonuklease Sau3A partiell hydrolisiert. Die Restriktionsfragmente wurden durch Elektrophorese auf einem Agarosegel aufgetrennt und die Fragmente mit einer Größe von 3 bis 8 Kilobasen (KB) wurden isoliert.

Die isolierten und größenselektierten DNA-Fragmente aus Bacillus alcalophilus HA1 wurden mit Vektor-DNA des Plasmids pUB 110 (Gewinnung wie in Beispiel 7 beschrieben) in vitro neukombiniert.

Hierzu wurde das Plasmid pUB110 zunächst mit der Restriktionsendonuklease BamHI restringiert und anschließend mit alkalischer Phosphatase aus Kälberdarm dephosphoryliert. Anschließend wurden 2 μg der restringierten und dephosphorylierten Vektor-DNA mit 8 μg der Bacillus alcalophilus DNA-Fragmente in einem Gesamtvolumen von 100 μl mit T4-DNA Ligase 24 h bei 16 ° C inkubiert.

Mit der erhaltenen in vitro neukombinierten DNA wurden Protoplasten des Stammes Bacillus subtilis BD224 nach der von S. Chang und N. Cohen (1979, Mol. Gen. Genet. 168: 111-115) beschriebenen Methode transformiert. Die Transformanten wurden auf Platten mit Neomycin selektiert und anschließend auf Magermilchagar überführt. Unter 13800 untersuchten Transformanten wurde eine gefunden, die durch Proteolyse des Magermilchagars einen deutlich größeren Hof bildete. Aus diesem Klon wurde die Plasmid-DNA nach Maniatis et al. isoliert. Das in diesem Plasmid enthaltene klonierte Fragment aus der B. alcalophilus-DNA hatte eine Größe von 4,1 KB und enthielt die vollständige Information für die hochalkalische Protease aus Bacillus alcalophilus HA1.

Zur Vereinfachung des weiteren Verfahrens wurde das 4,1 KB große DNA-Fragment zunächst in der Größe reduziert. Hierzu wurden die auf dem DNA-Fragment befindlichen Erkennungsstellen für Restriktionsendonukleasen durch Schneiden des Plasmids mit verschiedenen Restriktionsendonukleasen und Auftrennung der Fragmente der restringierten DNA durch Elektrophorese auf einem Agarosegel ermittelt. Es wurde ein 2,3 KB großes, durch Schneiden mit den Restriktionsendonukleasen AvaI und HindIII erhältliches DNA-Fragment ermittelt, welches die vollständige Information für die hochalkalische Protease aufwies und welches für das weitere Verfahren verwendet wurde. Hierzu wurde das obige Plasmid mit dem 4,1 KB-Fragment mit den Restriktionsendonukleasen AvaI und HindIII restringiert. Das 2,3 KB große DNA-Fragment wurde isoliert und mit dem Vektor pUB131 (Gewinnung wie in Beispiel 7 beschrieben), der zuvor ebenfalls mit AvaI und HindIII geschnitten wurde, ligiert.

Das erhaltene Plasmid, das die Bezeichnung pCLEAN4 erhielt, wurde in den Stamm B. subtilis BD224 eingebracht. Die Transformanten waren in der Lage, die hochalkalische Protease auszuscheiden, was zeigt, daß das AvaI/HindIII-Fragment das vollständige Strukturgen für die hochalkalische Protease aus B. alcalophilus HA1 enthält. Die Restriktionskarte des Plasmids pCLEAN4 ist in Fig. 2 wiedergegeben.

Beispiel 2

Sequenzierung von Strukturgenen am Beispiel der hochalkalischen Protease aus B. alcalophilus Zur Herstellung von Einzelstrang-DNA des Proteasestrukturgens wurde das Plasmid pCLEAN4 mit den

Restriktionsendonukleasen AvaI und HindIII geschnitten und das etwa 2,3 KB große AvaI/HindIII-DNA-Fragment (Proteasestrukturgen) in den Phagemiden pBS (+) oder pBS (-) eingebracht. Die Nukleotidsequenz des in den isolierten Einzelstrang-Phagemiden enthaltenen Proteasegens wurde nach der Dideoxy-Kettenterminator-Methode von Sanger et al. (1977, Proc. Natl. Acad. Sci. USA 74:5463) und der Methode der basenspezifischen chemischen Spaltung des DNA-Einzelstranges nach Maxam et al. (1980, in Methods in Enzymology, Grossmann L., Moldave K., eds., Academic Press Inc., New York und London, Vol. 65, 499) bestimmt. Die ermittelte Nukleotidsequenz und die zugeordnete Aminosäurensequenz der Protease sind in Fig. 1 wiedergegeben. Der Start für die Aminosäurensequenz der reifen hochalkalischen Protease in Position 1190 der Nukleotidsequenz wurde durch Aminosäurensequenzierung des N-terminalen Endes der hochalkalischen Protease bestimmt.

Beispiel 3

Herstellung mutierter DNA-Sequenzen durch gerichtete Mutagenese Die gerichteten Mutationen wurden in DNA-Teilsequenzen des Proteasestrukturgens mit der von Kunkel, T.A. (1985, Proc.Natl.Acad.Sci.USA 82:488-492) beschriebenen "primer extension" Technik durchgeführt. Hierzu wurden die Plasmide pCLMUTN1 (Herstellung wie in Beispiel 4 beschrieben) und pCLMUTC1 (Herstellung wie in Beispiel 5 beschrieben), die zunächst wie nachfolgend beschrieben in ihre uracylierten, einzelsträngigen Analoga umgewandelt wurden, eingesetzt. Die Ausgangsvektoren pCLMUTN1 und pCLMUTC1 enthalten nicht die gesamte DNA-Sequenz des Proteasestrukturgens aus B. alcalophilus HA1, sondern nur die N-terminale Hälfte (pCLMUTN1) oder die C-terminale Hälfte (pCLMUTC1) desselben.
Diese Vektoren sind als Abkömmlinge eines Phagemiden in gewissem Umfange zur Bildung von Einzelstrang-Vektor-DNA befähigt, die unter den hier gegebenen Bedinungen aus dem zur Replikation dienenden Wirtsorganismus ausgeschleust und isoliert werden konnte.
Jeder dieser Vektoren wurde nach Maniatis et al. (S. 250 bis 251) mit Hilfe der $CaCl_2$ Methode in E. coli CJ236 als Wirtsorganismus eingebracht.
Da das Bakterium E. coli CJ236 (Uracil-N-Glycosylase-Mangelmutante) bei der Replikation von Vektoren statt Thymin das Nukleotid Uracil in die DNA-Sequenz des Vektors einbaut, wurden durch Kultivierung der vorstehenden Transformanten die Uracil-haltigen Analoga des Vektors pCLMUTN1 oder pCLMUTC1 erhalten. Diese Uracil-enthaltenden Vektoren sind von den gewöhnlichen Thymin-enthaltenden Vektoren bei in vitro-Reaktionen nicht unterscheidbar. Der Uracil-Gehalt in der Vektor-DNA stört in vitro DNA-Synthesen nicht, da Uracil weder in vitro noch in vivo mutagen ist und Uracil gleichermaßen wie Thymin codiert. Uracylierte Vektoren lassen sich vorteilhaft für die nachfolgenden in vitro Reaktionen der gerichteten Mutagenese einsetzen. Nach Beendigung der Reaktionen kann der Uracil-haltige DNA-Einzelstrang, der als Matrize zur Erzeugung mutierter DNA-Stränge (Vektoren) diente, durch Behandlung mit Uracil-N-Glycosylase beseitigt werden, ohne daß es einer phänotypischen Selektion von Mutanten bedarf. Die Glycosylase-Behandlung kann sowohl mit dem isolierten Enzym als auch mit einem durch Vektor-DNA transformierten E. coli-Stamm mit Uracil-N-Glycosylase-Aktivität durchgeführt werden.
Die für die gerichtete Mutagenese als Matrize benötigte, uracylierte einzelsträngige DNA der Vektoren pCLMUTN1 und pCLMUTC1 wurde hergestellt, indem mit einem der beiden Vektoren transformierte E. coli CJ236-Bakterien kultiviert wurden, die zusätzlich mit dem Helfer-Phagen M13K07 infiziert wurden.
Der Helfer-Phage selbst ist kaum vermehrungsfähig und zeigt keine störende Interaktion mit der Vektor-DNA der Vektoren pCLMUTN1 oder pCLMUTC1. Seine Aufgabe besteht in der Synthese von Hüllproteinen für die gebildete uracylierte einzelsträngige Vektor-DNA. Umhüllte Einzelstrang-Vektor-DNA wird aus dem Wirtsorganismus E. coli CJ236 ausgeschleust und kann aus dem Kulturmedium isoliert werden. Durch die Mithilfe des Helfer-Phagen wird die qualitative und quantitative Ausbeute an (hier an uracylierter) Einzelstrang-Vektor-DNA wesentlich erhöht.
Die isolierten, uracylierten DNA-Einzelstrang-Vektoren pCLMUTN1 oder pCLMUTC1 wurden mit den nach Beispiel 6 hergestellten, synthetischen Oligonukleotiden hybridisiert, die eine Mutationsstelle enthielten und gleichzeitig als Primer für die nachfolgende Ergänzung zum vollständigen DNA-Doppelstrang mit Mutation dienten.
Die Synthese des zweiten DNA-Stranges wurde unter Zugabe von Nukleotiden mit T4-DNA-Polymerase und die nachfolgende Ligation des neugebildeten Stranges mit T4-DNA-Ligase durchgeführt (Kunkel et al. 1987, Methods in Enzymol. 154, 367-382). Die gebildete Doppelstrang-Vektor-DNA wurde in E. coli MC1061 transformiert und die mutierten Vektoren wurden durch Überprüfen der entsprechenden unitären Restriktionsendonukleasen-Erkennungsstellen, die mit den synthetischen Oligonukleotiden eingeführt bzw. entfernt wurden, identifiziert.

13

Beispiel 4

Konstruktion des Vektors pCLMUTN1 Das in Beispiel 1 hergestellte Plasmid pCLEAN4 wurde mit AvaI geschnitten. Die überstehenden Enden ("sticky ends") wurden unter Zugabe der benötigten Nukleotide mit Hilfe des Klenow-Fragments der E. coli DNA-Polymerase I (Maniatis et al., S. 114) zum DNA-Doppelstrang aufgefüllt. Nach anschließender Restriktion dieser DNA mit XbaI wurde das N-terminale 1618 Basenpaar (BP) umfassende Fragment des Proteasegens isoliert und in die SmaI/XbaI-Stelle von pBS kloniert. Der resultierende Vektor erhielt die Bezeichnung pCLMUTN1. Die Restriktionskarte dieses Vektors ist in Fig. 4 wiedergegeben.

Beispiel 5

Kontruktion des Vektors pCLMUTC1 Das in Beispiel 1 hergestellte Plasmid pCLEAN4 wurde mit den Restriktionsendonukleasen XbaI und Asp718 geschnitten. Das 658 BP umfassende XbaI/Asp718-Doppelstrang-DNA-Fragment, welches die C-terminale Hälfte des Proteasestrukturgens umfaßt, wurde in die XbaI/Asp718-Stelle von pBS kloniert. Der resultierende Vektor erhielt die Bezeichnung pCLMUTC1. Die Restriktionskarte des Vektors ist in Fig. 5 wiedergegeben.

Beispiel 6

Synthese künstlicher Oligonukleotide für die gerichtete Mutagenese

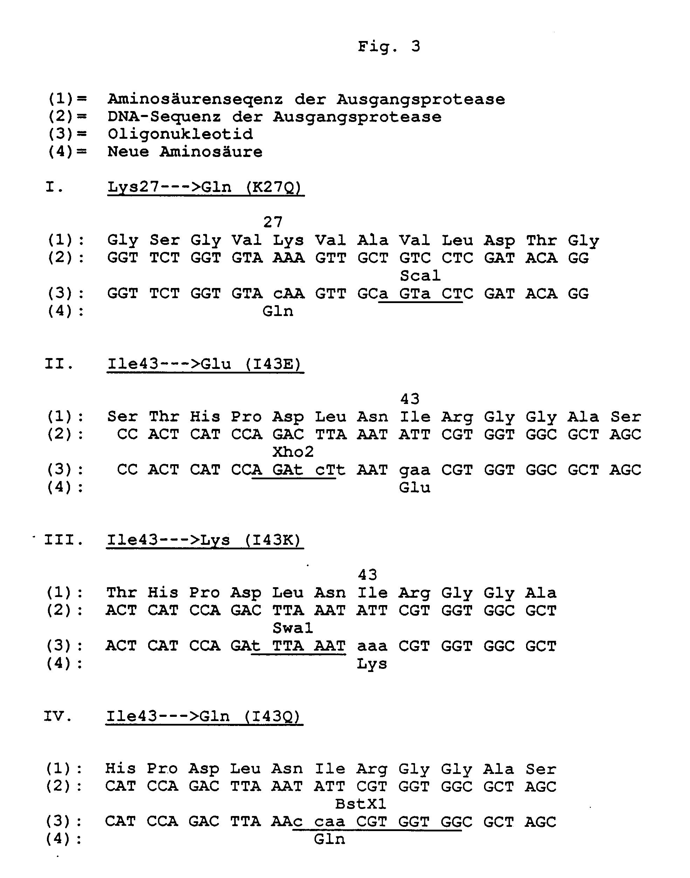

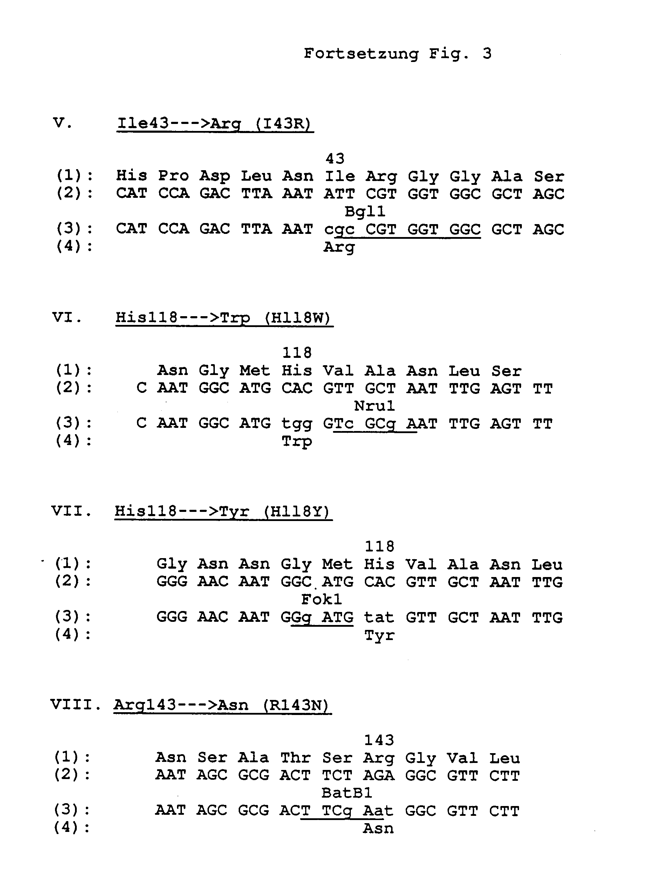

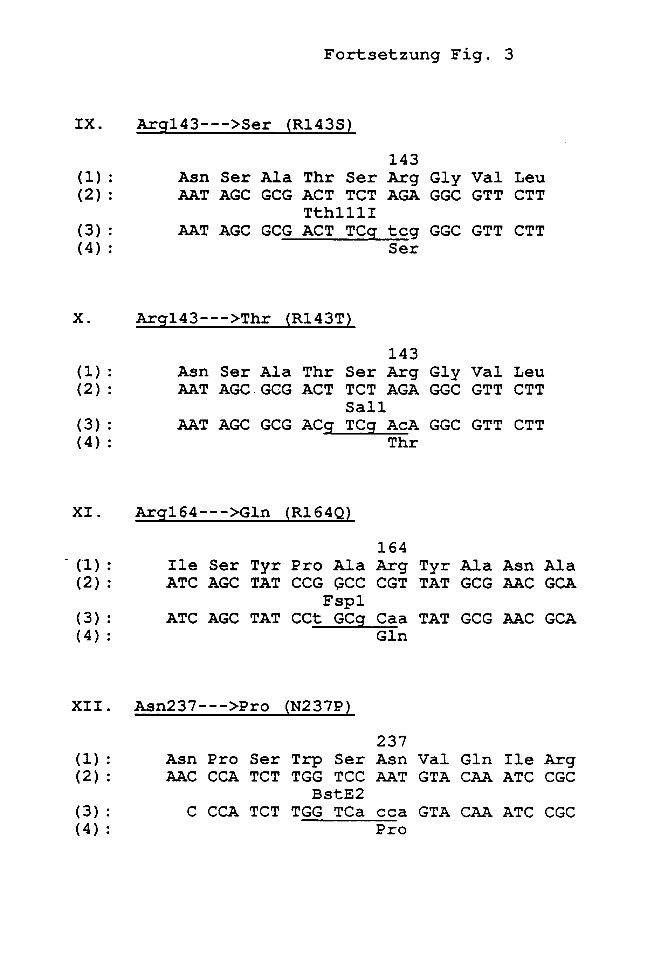

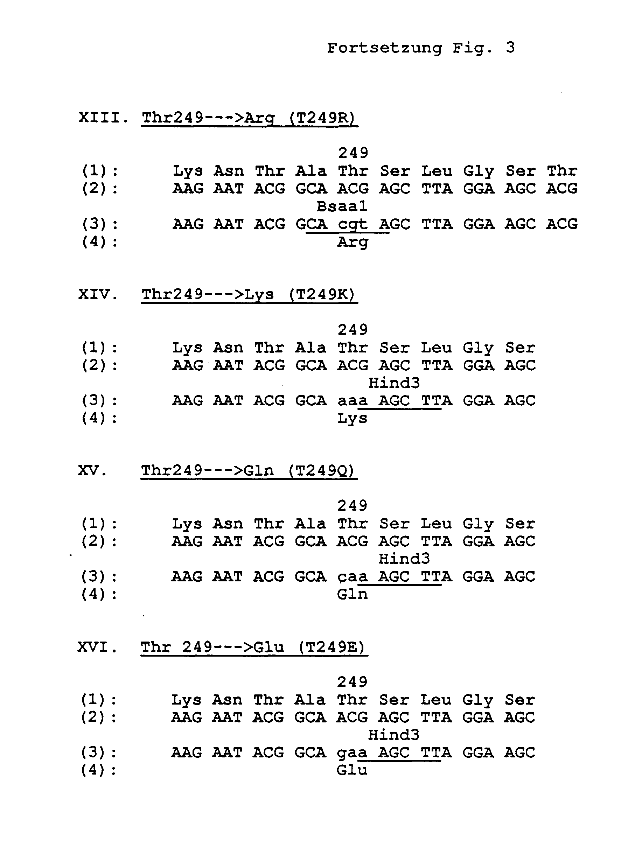

Synthetische Oligonukleotide wurden nach Beaucage S.L. und Caruthers M.H. (1981, Tetrahedron Letters 22: 1859 - 1862) mit $\beta$-Cyanoethyl-phosphoramidit in einem Cyclone Synthetiser (Biosearch) hergestellt. Die erhaltenen Oligonukleotide wurden gereinigt durch Elution aus Polyacrylamidgelen und anschließende Entsalzung mit Hilfe von Sephadex-G25-Säulen. Beispiele für die synthetisierten Nukleotidsequenzen und deren Eigenschaften sind in Fig. 3 wiedergegeben. Die Sequenzen der synthetischen Oligonukleotide, die im Verfahren nach Beispiel 3 zur Einführung der Mutationen in das Proteasegen dienten, waren so gewählt, daß sie die nachfolgenden Bedingungen erfüllten.

- Die DNA-Sequenz der synthetischen Oligonukleotide war zur entsprechenden Sequenz des Proteasegens noch soweit komplementär, daß ausreichende Hybridisierungsfähigkeit derselben sichergestellt war.
- Austausch eines oder mehrerer Nukleotide innerhalb des Codons, welches für die auszutauschende Aminosäure codiert, durch andere Nukleotide, so daß dieses mutierte Codon nunmehr für eine Aminosäure mit einem voluminö seren und/oder ionischen Aminosäurerest codierte (Mutationen). Für die neue Aminosäure wurde dasjenige Codon eingesetzt, welches im Proteasegen für die entsprechende Aminosäure am häufigsten vorgefunden wurde.
- Austausch von weiteren Nukleotiden innerhalb anderer Codons, so daß die ursprüngliche Codierung der Aminosäure zwar erhalten blieb, aber dadurch im Proteasegen vorkommende Erkennungssequenzen für Restriktionsendonukleasen entfernt oder neue erzeugt wurden. Diese dienten im Verfahren nach Beispiel 3 zur Erleichterung des Screenings nach den Vektoren mit den mutierten DNA-Sequenzen für die neuen hochalkalischen Proteasen.

Beispiel 7

Isolierung und Reinigung des Plasmids pUB110 und Konstruktion des Vektors pUB131 Aus dem Stamm Bacillus subtilis BD366 wurde nach der Methode von T.J. Gryczan et al. (1978, J. Bacteriol. 134: 318-329) das Plasmid pUB110 isoliert und anschließend nach Maniatis et al. (S. 93) über Cäsiumchlorid-Dichtegradientenzentrifugation gereinigt. Der Vektor pUB110 enthält eine nur einmal vorkommende Restriktionsstelle für die Restriktionsendonuklease BamHI und als Marker eine DNA-Sequenz, die für Antibiotikaresistenz gegenüber Neomycin codiert, sowie für die Replikation in Bacillus-Spezies benötigte DNA-Sequenzen ("origin of replication").

Das vorstehend erhaltene Plasmid pUB110 wurde mit EcoRI und BamHI restringiert. Das kleinere Fragment (790 BP) wurde ersetzt durch einen aus 67 Basenpaaren bestehenden Polylinker, der zuvor als EcoRI/BglII-Fragment aus dem Vektor M13tg131 isoliert worden war. Der so erhaltene Vektor mit der Bezeichnung pUB131 ist somit ein Abkömmling von pUB110, bei dem das etwa 0,8 KB große EcoRI/BamHI Fragment deletiert und dafür eine Polyklonierungsstelle eingebaut wurde.

Beispiel 8

Konstruktion der Vektoren pUBC131 und pUBC132 Das Plasmid pUC18 wurde mit AatII und PvuII geschnitten. Das 1990 Basenpaar große Fragment mit dem β-Lactamase-Gen und dem E. coli "origin of replication" wurde isoliert. Die überstehenden Enden ("sticky ends") wurden unter Zugabe der benötigten Nukleotide mit Hilfe des Klenow-Fragmentes der E. coli DNA-Polymerase I (Maniatis et al., S. 114) zum DNA-Doppelstrang aufgefüllt. Das Fragment wurde anschließend in die SnaBI-Stelle des nach Beispiel 7 erhaltenen Vektors pUB131 eingebaut, wodurch der Vektor mit der Bezeichnung pUBC131 erhalten wurde.

Das 2187 BP EcoRI/BglII-Fragment des vorstehend erhaltenen Vektors pUBC131 wurde in die EcoRI/BamHI-Stelle von pBS (+) subkloniert, wobei man den Vektor mit der Bezeichnung pBSREPU erhielt. Anschließend wurde die Ncol- bzw. StyI-Erkennungsstelle, die in der DNA-Sequenz für das repU-Polypeptid im Vektor pBSREPU vorhanden ist (I. Maciag et al. 1988, Mol. Gen. Genet. 212: 232-240), durch gerichtete Mutagenese eliminiert, indem die Nukleotidsequenz CCA TGG durch die Nukleotidsequenz CCG TGG (beide Nukleotidsequenzen codieren für die Aminosäurefolge Tryptophan-Prolin) ersetzt wurde. Die Durchführung war analog der Verfahrensweise des Beispiels 3. Hierzu wurde uracylierte einzelsträngige DNA des Vektors pBSREPU als Matrize für die gerichtete Mutation zur Eliminierung der Ncol- bzw. StyI-Erkennungsstelle hergestellt. Anschließend wurde diese Matrize analog zur im Beispiel 3 beschriebenen "primer extension"-Technik unter Verwendung des folgenden synthetischen Oligonukleotids (hergestellt und gereinigt analog zum Verfahren zur Herstellung der synthetischen Oligonukleotide des Beispiels 6)

<div align="center">

**NcoI/StyI**

**Pro Trp**

**Originäre repU-Sequenz     : AAA GTG AGA <u>CCA TGG</u> AGA GAA AA**

**Synthetische repU-Sequenz: AAA GTG AGA CCg TGG AGA GAA AA**

</div>

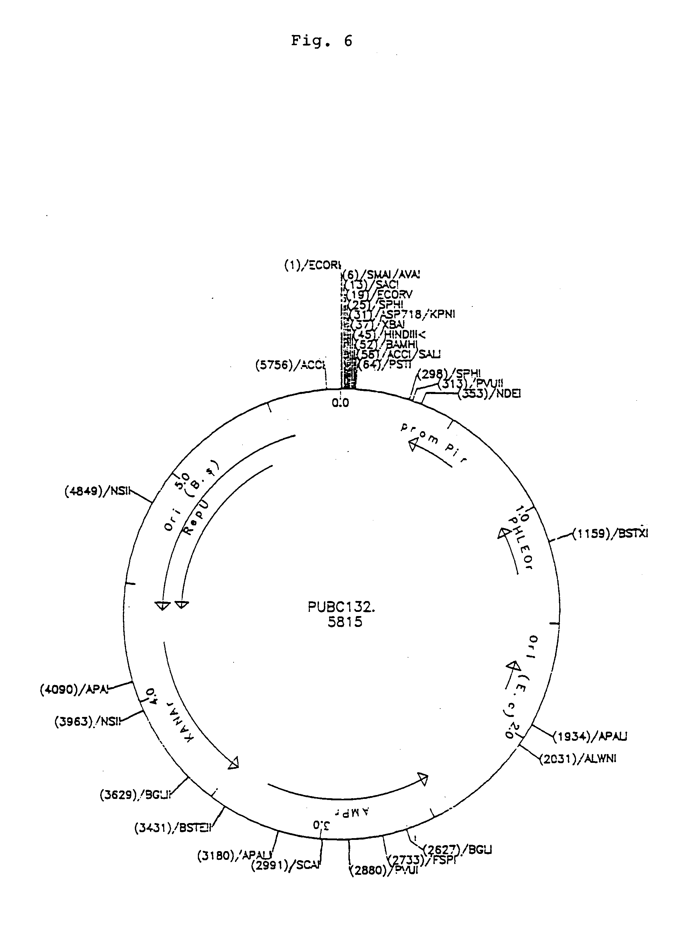

zum DNA-Doppelstrang-Vektor ergänzt und durch Transformation und Kultivierung von E. coli MC1061 die nunmehr Ncol- bzw. StyI-Erkennungsstellen-freien Vektoren isoliert. Das 1726 BP EcoRI/ApaI-Fragment des isolierten Vektors wurde in die EcoRI/ApaI-Stelle von pUBC131 eingeführt. Der neue Vektor, dessen Restriktionskarte in Fig. 6 wiedergegeben ist, erhielt die Bezeichnung pUBC132.

Beispiel 9

Konstruktion des Plasmids pAL1P

Das Plasmid pAL1P wurde hergestellt durch Ligation der folgenden drei Elemente:
- das 2218 Basenpaar große AvaI/NcoI-Fragment von pCLEAN4; das Fragment enthält den Promotor und die Prepro-Region der hochalkalischen Ausgangsprotease.
- der folgende synthetische Linker mit einzelnen Erkennungsstellen für die Restriktionsendonukleasen Ncol, XbaI und Asp718, die die Einführung der mutierten N-terminalen bzw. C-terminalen Hälften des Proteasegens aus den mutierten Vektoren pCLMUT1 bzw. pCLMUTC1 oder die Einführung des gesamten Gens der Ausgangsprotease aus dem Plasmid pCLEAN4 ermöglichen;

<div align="center">

**5' - CCATGGTCTAGAGGTACCA - 3'**

**3' - CAGATCTCCATGGTTCGAA - 5'**

**^          ^          ^          ^**

**NcoI   XbaI   Asp718**

**HindIII**

</div>

Der vorstehende doppelsträngige synthetische Linker mit überstehenden 5'-Enden wurde hergestellt, indem man zunächst die beiden Einzelstrang-DNA-Sequenzen analog zur Synthese der synthetischen Oligonukleotide in Beispiel 6 jeweils für sich herstellte und reinigte. Die so erhaltenen Einnzelstränge wurden nachfolgend miteinander zum Doppelstrang hybridisiert.

- das 5776 Basenpaar große AvaI/HindIII-Fragment aus dem in Beispiel 8 hergestellten Vektor pUBC132; dieses Fragment enthält DNA-Sequenzen zur Replikation und selektierbare Marker in E. coli, sowie DNA-Sequenzen zur Replikation und selektierbare Marker in B. subtilis, B. licheniformis und B. alcalophilus.

Die Konstruktion des Vektors pAL1P wurde in E. coli MC1061 durchgeführt und der Vektor aus Ampicillin-resistenten E. coli-Transformanten isoliert. Die Restriktionskarte des erhaltenen Vektors ist in Fig. 7 wiedergegeben.

Beispiel 10

Konstruktion der Plasmide pAL1N und pAL1C

Die Konstruktion der Vektoren pAL1N und pAL1C wurde in E. coli MC1061 durchgeführt, wobei die Vektoren aus Ampicillin-resistenten E. coli-Transformanten isoliert wurden.

Das Plasmid pAL1N wurde konstruiert, indem zunächst der in Beispiel 1 erhaltene Vektor pCLEAN4 mit den Restriktionsendonukleasen NcoI und XbaI geschnitten und das erhaltene NcoI/XbaI-Fragment anschließend in die NcoI/XbaI-Stelle des Vektors pAL1P (hergestellt nach Beispiel 9) kloniert wurde. Der hergestellte Vektor enthielt den N-terminalen Teil der DNA-Sequenz, die für das reife Enzym codiert, und die regulatorischen Elemente für die Transkription und Translation der hochalkalischen Protease, sowie die Signal-Sequenz und die Processing-Sequenz.

Das Plasmid pAL1C wurde konstruiert, indem zunächst der in Beispiel 1 erhaltene Vektor pCLEAN4 mit den Restriktionsendonukleasen XbaI und Asp718 geschnitten und das erhaltene XbaI/Asp718-Fragment in die XbaI/Asp718-Stelle des Vektors pAL1P (hergestellt nach Beispiel 9) kloniert wurde. Der hergestellte Vektor enthielt den C-terminalen Teil der DNA-Sequenz, die für die reife Protease codiert, und die regulatorischen Elemente für die Transkription und Translation der hochalkalischen Protease, sowie die Signal-Sequenz und die Processing-Sequenz.

Beispiel 11

Kontruktion der Expressionsvektoren pAL1NC

Es wurden Expressionsvektoren mit Mutationen im C-terminalen Teil der Protease-DNA-Sequenz, Expressionsvektoren mit Mutationen im N-terminalen Teil der Protease-DNA-Sequenz und zu Vergleichszwecken auch Expressionsvektoren ohne Mutationen in der Protease-DNA-Sequenz hergestellt. Die Konstruktion der drei nachfolgend aufgeführten Expressionsvektoren wurde jeweils in E. coli MC1061 durchgeführt. Aus Ampicillin-resistenten E. coli-Transformanten wurden die Expressionsvektoren isoliert. Zur Expression mutierter und nicht-mutierter Proteasegene wurden die in diesem Beispiel hergestellten und isolierten Expressionsvektoren in B. subtilis BD224 eingebracht. Die Selektion erfolgte hier nach Neomycin- oder Phleomycin-Resistenz. Die Transformanten waren zur Produktion der durch Mutation stabilisierten (Transformanten mit Vektoren aus Abschnitten A., B. dieses Beispiels) bzw. der nicht-mutierten (Transformanten mit Vektor aus Abschnitt C. dieses Beispiels) hochalkalischen Protease befähigt. Die Restriktionskarte der hergestellten Vektoren des Typs pAL1NC ist in Fig. 8 wiedergegeben.

A. Expressionsvektor mit Mutationen im N-terminalen Teil der DNA-Sequenz der Protease

Der nach Beispiel 3 durch gerichtete Mutagenese erhaltene mutierte Vektor pCLMUTN1 wurde mit den Restriktionsendonukleasen NcoI und XbaI geschnitten. Das isolierte NcoI/XbaI-Fragment (mutierter N-terminaler Teil des Proteasestrukturgens mit den Mutationen K27Q, I43R, I43K, I43Q, I43E, H118W, H118Y) wurde in die NcoI/XbaI-Stelle des nach Beispiel 10 erhaltenen Plasmides pAL1C kloniert. Die erhaltenen Vektoren stellten vollständige Expressionsvektoren mit geeignetem Leseraster zur Expression der durch Mutation stabilisierten Proteasen dar.

B. Expressionsvektor mit Mutationen im C-terminalen Teil der DNA-Sequenz der Protease

Der nach Beispiel 3 durch gerichtete Mutagenese erhaltene mutierte Vektor pCLMUTC1 wurde mit den Restriktionsendonukleasen XbaI und Asp718 geschnitten. Das isolierte XbaI/Asp718-Fragment (mutierter C-terminaler Teil des Proteasestrukturgens mit den R143N, R143S, R143T, R164Q, N237P, T249R, T249K, T249Q, T249E) wurde in die XbaI/Asp718-Stelle des nach Beispiel 10 erhaltenen Plasmides pAL1N kloniert. Die erhaltenen Vektoren stellten vollständige Expressionsvektoren mit geeignetem Leseraster zur Expression der durch Mutation stabilisierten Proteasen dar.

c. Expressionsvektor mit der nicht-mutierten DNA-Sequenz der Ausgangsprotease

Der Expressionsvektor mit dem nicht-mutierten Ausgangsstrukturgen der Protease wurde erhalten, indem entweder das nicht-mutierte NcoI/XbaI-Fragment aus dem nach Beispiel 1 erhaltenen Plasmid pCLEAN4 in die NcoI/XbaI-Stelle von pAL1C (Beispiel 10) kloniert wurde; oder indem das XbaI/Asp718-Fragment aus dem nach Beispiel 1 erhaltenen Plasmid pCLEAN4 in die XbaI/Asp718-Stelle von pAL1N (Beispiel 10) kloniert wurde. Die so erhaltenen Vektoren waren vollständige Expressionsvektoren mit geeignetem Leseraster zur Expression der Ausgangsprotease.

Beispiel 12

Herstellung der durch Mutation stabilisierten hochalkalischen Proteasen und zu Vergleichszwecken auch der unstabilisierten Ausgangsprotease 50 ml Vorkulturmedium (20 g Tryptone, 10 g Hefe-Extrakt, 5 g NaCl, 75 g lösliche Stärke, 10 ml Maisquellwasser pro Liter) wurden mit einer Kolonie der zu testenden Stämme (jeweils mit einem der nach Beispiel 11 hergestellten Vektoren pAL1NC transformierte B. subtilis BD224) beimpft. Die Kultur wurde für 16 h bei 37 °C und 250 Upm inkubiert. Mit 2,5 ml dieser Kultur wurden 50 ml Hauptkulturmedium (30 g Sojamehl, 90 g Kartoffelstärke, 10 g Na-Caseinat und 10 ml Maisquellwasser pro Liter) beimpft.

Die Hauptkultur wurde unter den gleichen Bedingungen wie die Vorkultur inkubiert. Nach 72 h wurden die Kulturen zentrifugiert. Die gebildeten Proteasen wurden aus den Kulturüberständen mit Aceton gefällt und anschließend wie folgt gereinigt: Kationenaustauscher Mono S, FPLC; Elution mit ansteigendem Gradient 20 mM bis 200 mM Ammoniumacetat, pH = 6.

Es wurden einerseits die Ausgangsprotease und andererseits die stabilisierten Proteasen der Aminosäurensequenz der Fig. 1 mit den Mutationen K27Q, I43K, I43Q, I43E, H118, H118Y, R143N, R143S, R143T, R164Q, N237P, T249R, T249K, T249Q und T249E gewonnen.

Beispiel 13

Waschleistungen der durch Mutation stabilisierten hochalkalischen Proteasen Die Waschleistungen erfindungsgemäß stabilisierter Proteasen wurde in Waschversuchen mit unterschiedlichen Waschmitteln und für unterschiedlich verschmutzte Gewebe ermittelt.

Beispiel 13a

Es wurde die Waschleistung der Proteasen durch Waschversuche am Testgewebe EY-PC (Eigelb/Tusche-Anschmutzung auf Polyester-Baumwolle-Mischgewebe - eigene Herstellung) in Laborwaschmaschinen (Typ: Polycolor) bestimmt. Hierzu wurden das Testgewebe mit einer wäßrigen Waschlauge, der die jeweils zu untersuchende Protease in einer Konzentration von 0,71 mg/l zugesetzt wurde, gewaschen. Als Waschmittel wurde eine für ein pulverförmiges Vollwaschmittel übliche Waschmittelformulierung mit üblichen Waschmittelinhaltsstoffen (18,4 % Zeolith, 7,3 % $Na_2CO_3$, 4,8 % lineares Alkylbenzolsulfonat, 3,3 % Nonionics, 3,3 % Seife, 0,1 % Entschäumer, 1,5 % Carboxymethylcellulose, 0,15 % optischen Aufheller, 3,8 % Natrium-disilikat, 25 % Perborat, 1,5 % TAED, 30,85 % $Na_2SO_4$) eingesetzt. Die Dosierung der Waschmittelformulierung in der Waschlauge betrug 6 g/l. Das verwendete Wasser hatte eine Wasserhärte von 15 °dH (°dH = deutsche Härte). Gewaschen wurde im Temperaturbereich von 15 °C bis 60 °C für 45 min (2 °C/min; 22,5 min Haltezeit bei 60 °C). Die enzymhaltige Waschlauge wirkte in einem rotierenden Probengefäß, das über ein Wasserbad entsprechend dem Temperaturprogramm gesteuert wurde, auf das Testgewebe ein. Nach dem Waschvorgang wurde das Testgewebe zweimal mit Leitungswasser gespült und anschließend gebügelt.

Die Waschleistung wurde durch Messung der Reflektion des gewaschenen Testgewebes mit Hilfe eines Remissionsphotometers bestimmt. Zum Vergleich wurde ebenfalls die Reflektion des unter gleichen Bedingungen nur mit der Waschmittelformulierung ohne Proteasezusatz gewaschenen Testgewebes ermittelt.

In Waschversuchen mit der durch Austausch in der Aminosäurensequenz in Position N237P stabilisierten Protease wurde eine um 6,8 % höhere Reflektion des Testgewebes als bei ohne Proteasezusatz gewaschenen Testgewebe gemessen.

Beispiel 13b

Die Waschversuche wurden wie im Beispiel 13a beschrieben durchgeführt. Als Waschmittel wurde eine enzymfreie von Waschmittelherstellern beziehbare Waschmittelformulierung eingesetzt, wie sie für ein pulverförmiges Kompaktvollwaschmittel üblich ist. Die Dosierung der Waschmittelformulierung in der Waschlauge betrug 3 g/l. In diesen Versuchen wurde im Temperaturbereich 15 °C bis 40 °C für 45 min (2° C/min; 32,5 min Haltezeit bei 40 °C) gewaschen.

Als Testgewebe wurde ein mit Blut, Milch und Tusche angeschmutztes Polyester-Baumwolle-Mischgewebe (EMPA 117, bezogen von der Eidgenössischen Materialprüfungsanstalt, St. Gallen, Schweiz) verwendet.

Es wurden die in der nachfolgenden Tabelle 1 angegebenen Proteasen auf ihre Waschleistungen untersucht. Die angegebene Erhöhung der Reflektion des gewaschenen Testgewebes bezieht sich dabei auf unter gleichen Bedingungen ohne Proteasezusatz nur mit der Waschmittelformulierung gewaschenes Testgewebe.

Tabelle 1

| Waschleistungen erfindungsgemäß stabilisierter Proteasen auf Testgewebe EMPA 117 in Waschversuchen mit einer für ein pulverförmiges Kompaktvollwaschmittel üblichen Waschmittelformulierung | |
| --- | --- |
| Stabilisierte Protease durch Austausch in der Aminosäurensequenz in Position | Erhöhung der Reflektion des Testgewebes in Relation zu ohne Proteasezusatz gewaschenes Testgewebe in % |
| I43Q | 21,3 |
| I43K | 20,9 |
| N114R/N237P | 20,7 |

Beispiel 13c

Die Waschversuche wurden wie im Beispiel 13b beschrieben durchgeführt. Es wurde jedoch ein Testgewebe EY-PC (Eigelb/Tusche-Anschmutzungen auf Polyester-Baumwolle-Mischgewebe - eigene Herstellung) verwendet.

Es wurden die in der nachfolgenden Tabelle 2 angegebenen Proteasen auf ihre Waschleistungen untersucht. Wie bereits beschrieben, bezieht die angegebene Erhöhung der Reflektion des Testgewebes sich auf ohne Proteasezusatz unter gleichen Bedingungen nur mit der Waschmittelformulierung gewaschenes Testgewebe.

Tabelle 2

| Waschleistungen erfindungsgemäß stabilisierter Proteasen auf Testgewebe EY-PC in Waschversuchen mit einer für ein pulverförmiges Kompaktvollwaschmittel üblichen Waschmittelformulierung | |
| --- | --- |
| Stabilisierte Protease durch Austausch in der Aminosäurensequenz in Position | Erhöhung der Reflektion des Testgewebes in Relation zu ohne Proteasezusatz gewaschenes Testgewebe in % |
| I43Q | 11,0 |
| I43K | 7,7 |

Beispiel 13d

Die Waschversuche wurden wie im Beispiel 13a beschrieben durchgeführt. Als Waschmittel wurde eine enzymfreie von Waschmittelherstellern beziehbare Waschmittelformulierung eingesetzt, wie sie für ein Flüssigvollwaschmittel üblich ist. Die Dosierung der Waschmittelformulierung in der Waschlauge betrug 4 g/l. Gewaschen wurde im Temperaturbereich 15 °C bis 40 °C für 45 min (2 °C/min; 32,5 min Haltezeit bei 40 °C). Als Testgewebe wurde ein Testgewebe EY-PC (Eigelb/Tusche-Anschmutzungen auf Polyester-Baumwolle-Mischgewebe - eigene Herstellung) eingesetzt.

Es wurden die in der nachfolgenden Tabelle 3 angegebenen Proteasen auf ihre Waschleistungen untersucht. Die angegebene Reflektion des Testgewebes bezieht sich dabei auf ohne Proteasezusatz unter gleichen Bedingungen nur mit der Waschmittelformulierung gewaschenes Testgewebe.

Tabelle 3

| Waschleistungen erfindungsgemäß stabilisierter Proteasen auf Testgewebe EY-PC in Waschversuchen mit einer für ein Flüssigvollwaschmittel üblichen Waschmittelformulierung | |
| --- | --- |
| Stabilisierte Protease durch Austausch in der Aminosäurensequenz in Position | Erhöhung der Reflektion des Testgewebes in Relation zu ohne Proteasezusatz gewaschenes Testgewebe in % |
| T249K | 4,0 |
| T249E | 5,3 |
| I43E | 4,5 |

In allen durchgeführten Waschversuchen wurde für das unter Zusatz erfindungsgemäßer Proteasen gewaschene Testgewebe eine höhere Reflektion als bei für mit der Waschmittelformulierung gewaschenes Testgewebe gemessen. Dies belegt die sehr guten Wascheigenschaften erfindungsgemäß stabilisierter Proteasen, wobei auch unterschiedliche Proteinverschmutzungen problemlos beim Waschgang aus dem Gewebe entfernt wurden.

**Patentansprüche**

1. Gegen destabilisierende Einwirkungen von ionischen Tensiden stabilisiertes Enzym, dadurch gekennzeichnet, daß wenigstens eine der Aminosäuren, die sich in einem hydrophoben Oberflächenbereich des Enzyms oder in direkter Nachbarschaft zu diesem hydrophoben Oberflächenbereich befinden, durch eine andere Aminosäure ausgetauscht ist, wobei
    (a) eine hydrophobe Aminosäure, deren Aminosäurerest an der Bildung eines hydrophoben Oberflächenbereiches beteiligt ist, in dessen räumlicher Nachbarschaft sich wenigstens eine Aminosäure mit ionischem Aminosäurerest befindet, durch eine andere Aminosäure mit hydrophilem Aminosäurerest ausgetauscht ist, und/oder wobei
    (b) eine polare, ungeladene Aminosäure, die an einen hydrophoben Oberflächenbereich angrenzt, in dessen räumlicher Nachbarschaft sich wenigstens eine Aminosäure mit ionischem Aminosäurerest befindet, durch eine gegenüber der ursprünglichen Aminosäure sterisch anspruchsvollere Aminosäure, vorzugsweise durch eine sterisch anspruchsvollere Aminosäure mit einem langkettigeren hydrophilen Aminosäurerest, ausgetauscht ist, und/oder wobei
    (c) eine ionische Aminosäure, die sich in räumlicher Nachbarschaft zu einem hydrophoben Oberflächenbereich befindet, durch eine Aminosäure mit einem ungeladenen hydrophilen Aminosäurerest oder durch eine geladene Aminosäure, deren Aminosäurerest eine zum ionischen Tensid gleichgerichtete Ladung aufweist, ausgetauscht ist.

2. Stabilisiertes Enzym nach Anspruch 1, dadurch gekennzeichnet, daß die Enzyme in einem solchen hydrophoben Oberflächenbereich des Enzyms durch Austausch von Aminosäuren stabilisiert sind, der als Vertiefung, insbesondere als Mulde, Senke oder Loch ausgebildet ist.

3. Stabilisiertes Enzym nach einem der vorhergehenden Ansprüche, dadurch gekennzeichnet, daß die Stabilität des Enzyms gegen destabilisierende Einwirkung von anionischen Tensiden durch einen Aminosäureaustausch gemäß (a) oder (b) oder durch einen Austausch einer kationischen Aminosäure durch eine Aminosäure mit einem ungeladenen hydrophilen oder mit einem anionischen Aminosäurerest verbessert ist.

4. Stabilisiertes Enzym nach einem der vorhergehenden Ansprüche, dadurch gekennzeichnet, daß das Enzym eine Protease, Lipase, Amylase oder Cellulase, vorzugsweise eine Protease ist.

5. Stabilisiertes Enzym nach Anspruch 4, dadurch gekennzeichnet, daß die Protease eine Bacillus-Protease, vorzugsweise ein Subtilisin ist.

19

**6.** Stabilisiesrtes Enzym nach Anspruch 5, dadurch gekennzeichnet, daß das Subtilisin eine hochalkalische Protease mit einer Aminosäurensequenz mit mindestens 80 %, vorzugsweise mindestens 90 %, insbesondere aber mindestens 95 % Homologie zu der in Fig. 1 angegebenen Aminosäurensequenz ist.

**7.** Stabilisiertes Enzym nach Anspruch 6, dadurch gekennzeichnet, daß die hochalkalische Protease durch Austausch von Aminosäuren in wenigstens einer der Positionen 4, 14, 27, 39, 43, 47, 49, 77, 80, 89, 111, 117, 118, 127, 143, 161, 164, 165, 208, 232, 233, 235, 237, 249 oder 256, vorzugsweise 27, 43, 118, 143, 164, 237 oder 249, der Fig. 1 oder in einer der dazu homolgen Positionen stabilisiert ist.

**8.** Stabilisiertes Enzym nach Anspruch 7, dadurch gekennzeichnet, daß die hochalkalische Protease durch wenigstens einen der Aminosäureaustausche K27Q, I43R, I43K, I43Q, I43E, Y89Q, H118W, H118Y, R143N, R143S, R143T, R164Q, N237P, T249R, T249K, T249Q und T249E, mit den auf Fig. 1 bezogenen Positionen, oder einen hierzu homologen Aminosäureaustausch stabilisiert ist.

**9.** Verfahren zur Verbesserung der Stabilität von Enzymen gegen destabilisierende Einwirkungen von ionischen Tensiden, dadurch gekennzeichnet, daß man wenigstens eine der Aminosäuren, die sich in einem hydrophoben Oberflächenbereich des Enzyms oder in direkter Nachbarschaft zu diesem hydrophoben Oberflächenbereich befinden, durch eine andere Aminosäure austauscht, wobei

(a) eine hydrophobe Aminosäure, deren Aminosäurerest an der Bildung eines hydrophoben Oberflächenbereiches beteiligt ist, in dessen räumlicher Nachbarschaft sich wenigstens eine Aminosäure mit ionischem Aminosäurerest befindet, durch eine Aminosäure mit hydrophilem Aminosäurerest ausgetauscht wird, und/oder wobei

(b) eine polare, ungeladene Aminosäure, die an einen hydrophoben Oberflächenbereich angrenzt, in dessen räumlicher Nachbarschaft sich wenigstens eine Aminosäure mit ionischem Aminosäurerest befindet, durch eine gegenüber der ursprünglichen Aminosäure sterisch anspruchsvollere Aminosäure, vorzugsweise durch eine sterische anspruchsvollere Aminosäure mit einem langkettigeren hydrophilen Aminosäurerest, ausgetauscht wird, und/oder wobei

(c) eine ionische Aminosäure, die sich in räumlicher Nachbarschaft zu einem hydrophoben Oberflächenbereich befindet, durch eine Aminosäure mit einem ungeladenen hydrophilen Aminosäurerest oder durch eine geladene Aminosäure, deren Aminosäurerest eine zum ionischen Tensid gleichgerichtete Ladung aufweist, ausgetauscht wird.

**10.** DNA-Sequenz, codierend für eine hochalkalische Protease mit einer Aminosäurensequenz, welche mindestens 80 %, vorzugsweise mindestens 90 %, insbesondere aber mindestens 95 % Homologie zu der in Fig. 1 angegebenen Aminosäurensequenz aufweist und sich von dieser in wenigstens einer der Positionen 4, 14, 27, 39, 43, 47, 49, 77, 80, 89, 111, 117, 118, 127, 143, 161, 164, 165, 208, 232, 233, 235, 237, 249 oder 256, vorzugsweise 27, 43, 118, 143, 164, 237 oder 249, der Fig. 1 oder in einer der dazu homologen Positionen dadurch unterscheidet, daß Positionen mit

(a) einer hydrophoben Aminosäure, deren Aminosäurerest an der Bildung eines hydrophoben Oberflächenbereiches beteiligt ist, in dessen räumlicher Nachbarschaft sich wenigstens eine Aminosäure mit ionischem Aminosäurerest befindet, durch eine andere Aminosäure mit hydrophilem Aminosäurerest ersetzt sind, und/oder daß Positionen mit

(b) einer polaren, ungeladenen Aminosäure, die an einen hydrophoben Oberflächenbereich angrenzt, in dessen räumlicher Nachbarschaft sich wenigstens eine Aminosäure mit ionischem Aminosäurerest befindet, durch eine gegenüber der ursprünglichen Aminosäure sterisch anspruchsvollere, vorzugsweise durch eine sterisch anspruchsvollere Aminosäure mit einem langkettigeren hydrophilen Aminosäurerest, ersetzt sind, und/oder daß Positionen mit

(c) einer ionischen Aminosäure, die sich in räumlicher Nachbarschaft zu einem hydrophoben Oberflächenbereich befindet, durch eine Aminosäure mit einem ungeladenen hydrophilen Aminosäurerest oder durch eine geladene Aminosäure, deren Aminosäurerest eine zum ionischen Tensid gleichgerichtete Ladung aufweist, ausgetauscht sind.

**11.** Wasch- und Reinigungsmittelzusammensetzungen, insbesondere enhaltend ionische Tenside, vorzugsweise anionische Tenside und ggf. weitere an sich übliche Wasch- und Reinigungsmittelkomponenten, dadurch gekennzeichnet, daß die Zusammensetzugen wenigstens ein stabilisiertes Enzym gemäß einem der Ansprüche 1 bis 8 enthalten.

20

Fig. 1

Sequenzprotokoll

ART DER SEQUENZ:            Nucleotid mit entsprechendem Protein
SEQUENZLÄNGE:               2280 Basenpaare

STRANGFORM:                 Einzelstrang
TOPOLOGIE:                  linear
ART DES MOLEKÜLS:           Genom-DNA

URSPRÜNGLICHE HERKUNFT:
ORGANISMUS:                 Bacillus alcalophilus
STAMM:                      HA1, DSM 5466

MERKMALE:
von 1192 bis 1998 BP reifes Protein


```
CTCGGGAAGC CGATTTGCTA CTGCATGTCG TCGATTATTC AAATGAACGC       50
CATCGCGAAA TGGCAAAGAC GACAAATGAA ACACTCCAGG CAATGGAAAT      100
CGATCGCCCG ATGATTTATG TTTACAACAA AATGGATCAA GTGAAAGACG      150
CGTTTCCTCA AGCGCATGGC ACGAGCTGTT TATATCAGCT AAGGCTAAAC      200
AAGGGCTTGA TTTATTAGCA CAGAAAATAG CAAGCTATGT TTTTCAAGAT      250
TTTGAAAAAC ATCTGTTCAT CATTCCTTAT CGTGACGGGG AGGCGGCTGC      300
TTATTTAAAC AACCATGCCC ATGTCCACAC ACAGCGTGCT GAGGAGGACG      350
GCTGGCATAT CGTTGCCGAT TTGCATGAAC GAGACTTAAA ACGGGTTGAA      400
AGCTACTGTG TTTCAAAAGA ACGATAATGA AAAAAGCCAT TTGAATGCTT      450
CTTGTTCAAA TGGCTTTTTG GCGACTATGG TAGACAGATG AACACTTGTT      500
TCGCTGTTTT ACGACAAAGA TCATCTTGCC TGTTACGCGT TTTTTAAATC      550
CGTTTTCGCA CGTTCAATTG TCGCCGAGTC GTACCAGTCG CTGTAAGTGA      600
GAATATGTTT AGAAAGCCGC GTATTTAAGC GCAGTCTTTT TCGTTCTGTA      650
CTGGCTGGTT TGTGGACAGT TTCCATACCC ATCAACCTCC TTTTATTTGT      700
AGCTTTCCCC ACTTGAAACC GTTTTAATCA AAAACGAAGT GAGAAGATTC      750
AGTTAACTTA ACGTTAATAT TTGTTTCCCA ATAGGCAAAT CTTTCTAACT      800
TTGATACGTT TAAACTACCA GCTTGGACAA GTTGGTATAA AAATGAGGAG      850
GGAACCGA ATG AAG AAA CCG TTG GGG AAA ATT GTC GCA           888
         Met Lys Lys Pro Leu Gly Lys Ile Val Ala
          -110                  -105

AGC ACC GCA CTA CTC ATT TCT GTT GCT TTT AGT TCA TCG        927
Ser Thr Ala Leu Leu Ile Ser Val Ala Phe Ser Ser Ser
  -100                -95                -90

ATC GCA TCG GCT GCT GAA GAA GCA AAA GAA AAA TAT TTA        966
Ile Ala Ser Ala Ala Glu Glu Ala Lys Glu Lys Tyr Leu
       -85                -80
```

Fortsetzung Fig. 1

```
ATT GGC TTT AAT GAG CAG GAA GCT GTC AGT GAG TTT GTA                1005
Ile Gly Phe Asn Glu Gln Glu Ala Val Ser Glu Phe Val
-75             -70             -65

GAA CAA GTA GAG GCA AAT GAC GAG GTC GCC ATT CTC TCT                1044
Glu Gln Val Glu Ala Asn Asp Glu Val Ala Ile Leu Ser
        -60             -55                 -50

GAG GAA GAG GAA GTC GAA ATT GAA TTG CTT CAT GAA TTT                1083
Glu Glu Glu Glu Val Glu Ile Glu Leu Leu His Glu Phe
                -45             -40

GAA ACG ATT CCT GTT TTA TCC GTT GAG TTA AGC CCA GAA                1122
Glu Thr Ile Pro Val Leu Ser Val Glu Leu Ser Pro Glu
    -35             -30                 -25

GAT GTG GAC GCG CTT GAA CTC GAT CCA GCG ATT TCT TAT                1161
Asp Val Asp Ala Leu Glu Leu Asp Pro Ala Ile Ser Tyr
        -20             -15

ATT GAA GAG GAT GCA GAA GTA ACG ACA ATG GCG CAA TCA                1200
Ile Glu Glu Asp Ala Glu Val Thr Thr Met Ala Gln Ser
-10             -5                  1

GTG CCA TGG GGA ATT AGC CGT GTG CAA GCC CCA GCT GCC                1239
Val Pro Trp Gly Ile Ser Arg Val Gln Ala Pro Ala Ala
    5               10              15

CAT AAC CGT GGA TTG ACA GGT TCT GGT GTA AAA GTT GCT                1278
His Asn Arg Gly Leu Thr Gly Ser Gly Val Lys Val Ala
            20              25

GTC CTC GAT ACA GGT ATT TCC ACT CAT CCA GAC TTA AAT                1317
Val Leu Asp Thr Gly Ile Ser Thr His Pro Asp Leu Asn
30              35              40

ATT CGT GGT GGC GCT AGC TTT GTA CCA GGG GAA CCA TCC                1356
Ile Arg Gly Gly Ala Ser Phe Val Pro Gly Glu Pro Ser
            45              50                  55

ACT CAA GAT GGG AAT GGG CAT GGC ACG CAT GTG GCC GGG                1395
Thr Gln Asp Gly Asn Gly His Gly Thr His Val Ala Gly
            60              65

ACG ATT GCT GCT TTA AAC AAT TCG ATT GGC GTT CTT GGC                1434
Thr Ile Ala Ala Leu Asn Asn Ser Ile Gly Val Leu Gly
    70              75              80

GTA GCG CCG AGC GCG GAA CTA TAC GCT GTT AAA GTA TTA                1473
Val Ala Pro Ser Ala Glu Leu Tyr Ala Val Lys Val Leu
            85              90

GGG GCG AGC GGT TCA GGT TCG GTC AGC TCG ATT GCC CAA                1512
Gly Ala Ser Gly Ser Gly Ser Val Ser Ser Ile Ala Gln
95              100             105

GGA TTG GAA TGG GCA GGG AAC AAT GGC ATG CAC GTT GCT                1551
Gly Leu Glu Trp Ala Gly Asn Asn Gly Met His Val Ala
        110             115             120
```

Fortsetzung Fig. 1

```
AAT TTG AGT TTA GGA AGC CCT TCG CCA AGT GCC ACA CTT              1590
Asn Leu Ser Leu Gly Ser Pro Ser Pro Ser Ala Thr Leu
            125                 130

GAG CAA GCT GTT AAT AGC GCG ACT TCT AGA GGC GTT CTT              1629
Glu Gln Ala Val Asn Ser Ala Thr Ser Arg Gly Val Leu
    135                 140                 145

GTT GTA GCG GCA TCT GGG AAT TCA GGT GCA GGC TCA ATC              1668
Val Val Ala Ala Ser Gly Asn Ser Gly Ala Gly Ser Ile
                150                 155

AGC TAT CCG GCC CGT TAT GCG AAC GCA ATG GCA GTC GGA              1707
Ser Tyr Pro Ala Arg Tyr Ala Asn Ala Met Ala Val Gly
160                 165                 170

GCT ACT GAC CAA AAC AAC AAC CGC GCC AGC TTT TCA CAG              1746
Ala Thr Asp Gln Asn Asn Asn Arg Ala Ser Phe Ser Gln
            175                 180                 185

TAT GGC GCA GGG CTT GAC ATT GTC GCA CCA GGT GTA AAC              1785
Tyr Gly Ala Gly Leu Asp Ile Val Ala Pro Gly Val Asn
                190                 195

GTG CAG AGC ACA TAC CCA GGT TCA ACG TAT GCC AGC TTA              1824
Val Gln Ser Thr Tyr Pro Gly Ser Thr Tyr Ala Ser Leu
    200                 205                 210

AAC GGT ACA TCG ATG GCT ACT CCT CAT GTT GCA GGT GCA              1863
Asn Gly Thr Ser Met Ala Thr Pro His Val Ala Gly Ala
                215                 220

GCA GCC CTT GTT AAA CAA AAG AAC CCA TCT TGG TCC AAT              1902
Ala Ala Leu Val Lys Gln Lys Asn Pro Ser Trp Ser Asn
225                 230                 235

GTA CAA ATC CGC AAT CAT CTA AAG AAT ACG GCA ACG AGC              1941
Val Gln Ile Arg Asn His Leu Lys Asn Thr Ala Thr Ser
        240                 245                 250

TTA GGA AGC ACG AAC TTG TAT GGA AGC GGA CTT GTC AAT              1980
Leu Gly Ser Thr Asn Leu Tyr Gly Ser Gly Leu Val Asn
                255                 260

GCA GAA GCG GCA ACA CGC TAATCAATAA AAAAAGCCTG TGCGGTTAAA        2028
Ala Glu Ala Ala Thr Arg
        265

GGGCACAGCG TTTTTTTGTG TATGAATCGA AAAAGAGAAC AGATCGCAGG          2078
TCTCAAAAAT CGAGCGTAAA GGGCTGTTTA AAGCTCTTTA CGCTCGCAGG          2128
TCTTATCGCT ATACAATGGA AAATTCACGT CTTTTGACTT TCATGGCATA          2178
TTTATTTAAG TATTCGTTTG CTTTTTCGTA CTCTCCGTTT TTCTGGTACC          2228
ATTGCGCCAG CTCAATTGCA TAGTGGACTG GTTCTTCTTT ATTATCAAGC          2278
TT                                                              2280
```

## Fig. 2

PCLEAN4.
6059

(1)/ECORI
(6)/AVAI
(106)/PVUI
(153)/MLUI
(539)/MLUI
(755)/HPAI
(929)/PVUI
(1207)/NCOI
(1235)/PVUII
(1242)/BSTXI
(1618)/XBAI
(1650)/ECORI
(2224)/ASP718/KPNI
(2276)/HINDIII<
(2283)/BAMHI
(2289)/SALI
(2295)/PSTI
(2544)/PVUII
(2584)/NDEI
(3390)/BSTXI
(3673)/SNABI
(3873)/BGLI
(4207)/NSII
(4334)/APAI
(4336)/BGLI
(5093)/NSII
(5543)/NCOI

Ori pUB
RepU
Pir 3'
Pir 5'
Protease
Mature
KANA
Bla

Fig. 3

(1)= Aminosäurenseqenz der Ausgangsprotease
(2)= DNA-Sequenz der Ausgangsprotease
(3)= Oligonukleotid
(4)= Neue Aminosäure

I.     <u>Lys27--->Gln (K27Q)</u>

```
                          27
(1): Gly Ser Gly Val Lys Val Ala Val Leu Asp Thr Gly
(2): GGT TCT GGT GTA AAA GTT GCT GTC CTC GAT ACA GG
                                     ScaI
(3): GGT TCT GGT GTA cAA GTT GCa GTa CTC GAT ACA GG
(4):                     Gln
```

II.     <u>Ile43--->Glu (I43E)</u>

```
                                          43
(1): Ser Thr His Pro Asp Leu Asn Ile Arg Gly Gly Ala Ser
(2):  CC ACT CAT CCA GAC TTA AAT ATT CGT GGT GGC GCT AGC
                          Xho2
(3):  CC ACT CAT CCA GAt cTt AAT gaa CGT GGT GGC GCT AGC
(4):                              Glu
```

III.     <u>Ile43--->Lys (I43K)</u>

```
                                  43
(1): Thr His Pro Asp Leu Asn Ile Arg Gly Gly Ala
(2): ACT CAT CCA GAC TTA AAT ATT CGT GGT GGC GCT
                     SwaI
(3): ACT CAT CCA GAt TTA AAT aaa CGT GGT GGC GCT
(4):                          Lys
```

IV.     <u>Ile43--->Gln (I43Q)</u>

```
(1): His Pro Asp Leu Asn Ile Arg Gly Gly Ala Ser
(2): CAT CCA GAC TTA AAT ATT CGT GGT GGC GCT AGC
                          BstX1
(3): CAT CCA GAC TTA AAc caa CGT GGT GGC GCT AGC
(4):                     Gln
```

25

Fortsetzung Fig. 3


V.   Ile43--->Arg (I43R)


```
                            43
(1):  His Pro Asp Leu Asn Ile Arg Gly Gly Ala Ser
(2):  CAT CCA GAC TTA AAT ATT CGT GGT GGC GCT AGC
                            Bgl1
(3):  CAT CCA GAC TTA AAT cgc CGT GGT GGC GCT AGC
(4):                       Arg
```


VI.  His118--->Trp (H118W)


```
                        118
(1):    Asn Gly Met His Val Ala Asn Leu Ser
(2):  C AAT GGC ATG CAC GTT GCT AAT TTG AGT TT
                            Nru1
(3):  C AAT GGC ATG tgg GTc GCg AAT TTG AGT TT
(4):              Trp
```


VII. His118--->Tyr (H118Y)


```
                            118
(1):    Gly Asn Asn Gly Met His Val Ala Asn Leu
(2):    GGG AAC AAT GGC ATG CAC GTT GCT AAT TTG
                        Fok1
(3):    GGG AAC AAT GGg ATG tat GTT GCT AAT TTG
(4):                    Tyr
```


VIII. Arg143--->Asn (R143N)


```
                        143
(1):    Asn Ser Ala Thr Ser Arg Gly Val Leu
(2):    AAT AGC GCG ACT TCT AGA GGC GTT CTT
                        BatB1
(3):    AAT AGC GCG ACT TCg Aat GGC GTT CTT
(4):                    Asn
```


26

Fortsetzung Fig. 3

IX.    <u>Arg143--->Ser (R143S)</u>

```
                                     143
(1):        Asn Ser Ala Thr Ser Arg Gly Val Leu
(2):        AAT AGC GCG ACT TCT AGA GGC GTT CTT
                            Tth111I
(3):        AAT AGC GCG ACT TCg tcg GGC GTT CTT
(4):                            Ser
```

X.    <u>Arg143--->Thr (R143T)</u>

```
                                     143
(1):        Asn Ser Ala Thr Ser Arg Gly Val Leu
(2):        AAT AGC GCG ACT TCT AGA GGC GTT CTT
                            SalI
(3):        AAT AGC GCG ACg TCg AcA GGC GTT CTT
(4):                            Thr
```

XI.    <u>Arg164--->Gln (R164Q)</u>

```
                                     164
(1):        Ile Ser Tyr Pro Ala Arg Tyr Ala Asn Ala
(2):        ATC AGC TAT CCG GCC CGT TAT GCG AAC GCA
                            FspI
(3):        ATC AGC TAT CCt GCg Caa TAT GCG AAC GCA
(4):                            Gln
```

XII.    <u>Asn237--->Pro (N237P)</u>

```
                                     237
(1):        Asn Pro Ser Trp Ser Asn Val Gln Ile Arg
(2):        AAC CCA TCT TGG TCC AAT GTA CAA ATC CGC
                            BstE2
(3):         C CCA TCT TGG TCa cca GTA CAA ATC CGC
(4):                            Pro
```

27

Fortsetzung Fig. 3

XIII.  Thr249--->Arg (T249R)

```
                                      249
(1):      Lys Asn Thr Ala Thr Ser Leu Gly Ser Thr
(2):      AAG AAT ACG GCA ACG AGC TTA GGA AGC ACG
                          Bsaa1
(3):      AAG AAT ACG GCA cgt AGC TTA GGA AGC ACG
(4):                      Arg
```

XIV.  Thr249--->Lys (T249K)

```
                                  249
(1):      Lys Asn Thr Ala Thr Ser Leu Gly Ser
(2):      AAG AAT ACG GCA ACG AGC TTA GGA AGC
                              Hind3
(3):      AAG AAT ACG GCA aaa AGC TTA GGA AGC
(4):                      Lys
```

XV.   Thr249--->Gln (T249Q)

```
                                  249
(1):      Lys Asn Thr Ala Thr Ser Leu Gly Ser
(2):      AAG AAT ACG GCA ACG AGC TTA GGA AGC
                              Hind3
(3):      AAG AAT ACG GCA caa AGC TTA GGA AGC
(4):                      Gln
```

XVI.  Thr 249--->Glu (T249E)

```
                                  249
(1):      Lys Asn Thr Ala Thr Ser Leu Gly Ser
(2):      AAG AAT ACG GCA ACG AGC TTA GGA AGC
                              Hind3
(3):      AAG AAT ACG GCA gaa AGC TTA GGA AGC
(4):                      Glu
```

28

Fig. 4

Fig. 5

Fig. 6

Fig. 7

Fig. 8

Fig. 9

Fig. 10

Fig. 11

Fig. 12